# EUROPÄISCHE PATENTSCHRIFT

(11) **EP 1 888 223 B1**
(45) Veröffentlichungstag und Bekanntmachung des Hinweises auf die Patenterteilung: **11.02.2009**
(21) Anmeldenummer: 06705797.6
(22) Anmeldetag: 16.01.2006
(51) Int. Cl.: B01J 13/22, B01J 13/02, A61K 9/127

(54) **VERFAHREN UND VORRICHTUNG ZUR EINKAPSELUNG VON STOFFEN IN LIPOSOMEN MIT FREI EINSTELLBAREM MEMBRANAUFBAU**
METHOD AND DEVICE FOR ENCAPSULATING MATERIALS IN LIPOSOMES COMPRISING A FREELY ADAPTABLE MEMBRANE STRUCTURE
PROCEDE ET DISPOSITIF D'ENCAPSULAGE DE SUBSTANCES DANS DES LIPOSOMES AYANT UNE STRUCTURE MEMBRANE LIBREMENT AJUSTABLE

(30) Priorität: 18.01.2005 DE 102005002469
(43) Veröffentlichungstag der Anmeldung: 20.02.2008
(73) Patentinhaber: Abnoba Heilmittel GmbH, 75177 Pforzheim (DE)
(72) Erfinder: LENEWEIT, Gero, 75223 Niefern-Öschelbronn (DE)
(74) Vertreter: Schneider, Uwe
(86) Internationale Anmeldenummer: PCT/DE2006/000058
(87) Internationale Veröffentlichungsnummer: WO 2006/076891

(56) Entgegenhaltungen:
- EP-A- 0 310 984
- WO-A-90/08535
- WO-A-99/36056
- WO-A-03/057191

## Beschreibung

Die Erfindung betrifft ein Verfahren und Vorrichtungen zur Einkapselung von Stoffen in Liposomen mit frei einstellbarem Membranaufbau gemäß Oberbegriff des Anspruches 1 bzw. gemäß Oberbegriff des Anspruches 21 bzw. 31.

Liposomen sind Membranbläschen, die einen flüssigen Innenraum umhüllen und in einem wässrigen Milieu kolloidal suspendiert sind. Die Membranen sind aus amphiphilen Lipiden, wie Phospholipiden und Glycolipiden aufgebaut, die eine Lipiddoppelschicht (Bischicht) formen. Häufig werden Cholesterol oder andere Sterole zur Herabsetzung der Fluidität in die Bischicht eingelagert. Liposomen können unilamellar, aber auch oligo- oder multilamellar vorliegen; ihre Größe kann, je nach Herstellungsverfahren, zwischen ca. 20 nm und größer als 1 µm liegen.

In Ergänzung zur üblichen wissenschaftlichen Verwendung der Bezeichnung "Liposom" (synonym mit dem Begriff "Vesikel") sind im Folgenden immer auch folgende kolloidchemischen Aggregate mit diesem Begriff angesprochen und können in erfindungsgemäßer Weise entsprechend wie Liposomen hergestellt, verarbeitet oder verwendet werden: Emulsionströpfchen in Mikro- und Nanometergröße, Polymerliposomen, Mizellen und Mischungen dieser Aggregatbildungen mit reinen Liposomen. Daher werden derartige kolloidchemische Aggregate im Weiteren immer auch dann mit einbezogen, wenn nur der Begriff Liposom benutzt wird.

Liposomen spielen in der modernen Pharmazie, Kosmetik und Lebensmitteltechnologie eine bedeutende Rolle als Transportvehikel für pharmazeutische Wirkstoffe, zur Verbesserung der Hautfeuchtigkeit oder Wirkstoffaufnahme bzw. für hochwertige Lebensmittelzusätze. Im Folgenden soll immer dann, wenn die vorstehend genannten verschiedenen Einsatzgebiete von Liposomen angesprochen werden, beispielhaft, aber nicht einengend auf die Bedeutung von Liposomen für die pharmazeutische Technologie eingegangen werden. Andere, hier nur beispielhaft genannte und und ohne Einschränkung genannte und für den Einsatzbereich der Erfindung relevante Gebiete können die Arzneistoff-Zuführung (drug delivery), die synthetische Chemie allgemein, nanoskalige Reaktionskammern sowie allgemeine technologische Entwicklungen in den Bereichen Energie, Optik, Elektronik, Mikrofluidik, Kolloidchemie, Biosensorik oder artverwandte Gebiete sein, in denen Liposome und kolloidchemische Aggregate Verwendung finden können oder entsprechende Herstellungsverfahren zum Einsatz kommen können.

Liposomen können mit einer Polyethylenglykolschicht überzogen (man spricht dann von PEGyliert) sein, die sie sterisch schützt und eine zusätzliche Hydrathülle bildet [siehe hierzu Papisov, M.I. (1998) Theoretical considerations of RES-avoiding liposomes: Molecular mechanics and chemistry of liposome interactions, Advanced Drug Delivery Reviews, 32, 119-138.]. Hydrophile Wirkstoffe können in Liposomen eingekapselt, lipophile Stoffe dagegen in die Bischicht eingebaut werden. Die Pharmakokinetik wird dann von der Natur der Liposomen beeinflußt, kaum mehr von der Natur des Wirkstoffs. Durch den Aufbau der Liposomen (Membranzusammensetzung, PEGylierung, Reduzierung der Größe) kann das so genannte "drug targeting" optimiert werden, was z.B. für die Tumortherapie eine maximale Anreicherung der Wirkstoffe im Tumorgewebe bedeutet. Aus diesem Grund werden heute viele bekannte Chemotherapeutika als liposomale Formulierungen (z.B. DaunoXome^{®}, Caelyx^{®}) appliziert, da damit die therapeutischen Wirkungen erhöht, die Nebenwirkungen aber erniedrigt sind.

Die bisherigen Herstellungstechniken von Liposomen beruhen im Wesentlichen auf zwei alternativen Verfahren:
□ den Homogenisierungsmethoden, bei denen zunächst Rohliposomen durch die Filmmethode erzeugt [Bangham, A.D., Standish, M.M. & Watkins, J.C. (1965) Diffusion of univalent ions across the lamellae of swollen phospholipids, J. Mol. Biol., 13, 238-252.] und diese anschließend mechanisch auf eine einheitliche Größe gebracht werden; und
□ der Detergensdialyse.

Allen bisher entwickelten Herstellungstechniken ist gemeinsam, daß bei der Einkapselung der wässrige Innenraum identisch mit dem Außenraum ist. Bei einer üblichen Einkapselungskapazität von ca. 2% bedeutet dies, daß 98% des Wirkstoffs nachträglich aus dem Außenraum entfernt werden müssen. Eine nachträgliche Beladung der Liposomen mit thermischen Methoden oder pH-Gradienten ist nur für bestimmte Wirkstoffe möglich und bringt andere Nachteile mit sich (wie z.B. verringerte Lagerstabilität, zusätzliche Bearbeitungsschritte).

Eine zweite Unzulänglichkeit der bisher entwickelten Herstellungstechniken ist die Uniformität der Membran, d.h. die Innen- und Außenseite der Bischicht sind identisch. Bei allen biogenen Membranen sind die Innen- und Außenseite der Membran jedoch stets unterschiedlich, sowohl in ihrer Lipid- als auch in ihrer Proteinkomposition. Die Asymmetrie biologischer Membranen hat eine wichtige physiologische Funktion für verschiedene zelluläre Erkennungs- und Transportmechanismen. Entsprechend wäre die uneingeschränkte Manipulierbarkeit der Zusammensetzung der Innen- und Außenseite von Liposomenmembranen wünschenswert, z.B. für das Anlagern von Wirkstoffen auf der Innenseite und von sterischen Schutzschichten oder Rezeptoren auf der Außenseite, was derzeit nur durch nachträgliche Bearbeitungsschritte eingeschränkt möglich ist.

Die Möglichkeit, Monoschichten zu einer Bischicht zu synthetisieren und durch Zentrifugalkräfte zu Liposomen abzuschnüren, ist in der wissenschaftlichen Literatur bereits an anderer Stelle zu finden [Träuble, H. & Grell, E. (1971): The formation of a-symmetrical spherical lecithin vesicles, Neuroscience Research Program Bulletin, 9, 373-380].

Unter dem Begriff Monoschicht werden üblicherweise monomolekulare Schichten grenzflächenaktiver Substanzen verstanden. In Ergänzung zu dieser Definition sind im Weiteren unter dem Begriff Monoschicht immer auch alle Substanzen und Substanzklassen angesprochen und vom Schutz mit umfasst, die in der Lage sind, dünne Schichten zu bilden, wie z. B. Polymere und Proteine, und insbesondere auch, wenn diese Substanzklassen und Substanzen ggf. mehr als eine einzige Molekülschicht umfassen oder umfassen können. Analog ist unter Bischicht im weiteren Kontext immer auch die Zusammenfügung zweier Monoschichten in dem obigen Sinne zu verstehen.

Entsprechende Verfahren zur Herstellung von Liposomen für pharmazeutische Zwecke im Rahmen der Misteltherapie sind aus der EP 0288603 B1 sowie der EP 0310984 B1 bekannt. Mit dem Verfahren der EP 0288603 B1 werden Pflanzenextrakte, insbesondere Mistelextrakte durch Pressverfahren hergestellt, bei denen das Pflanzenzellgewebe bei hohem Druck durch einen 10 µm engen Spalt gepresst und dabei homogenisiert werden. Die Membranen der Zellwände und die Zellorganellen werden dabei aufgebrochen und ihre Bruchstücke formen sich dabei zu kleinen Hohlbläschen mit wäßrigem Inhalt in wässriger Umgebung um [Koehler, R., Laue, H.B.v. & Leneweit, G. (2001): Veränderungen von Mistelextrakten durch ein pharmazeutisches Strömungsverfahren, in: R. Scheer, R. Bauer, H. Becker, P.A. Berg & V. Fintelmann (Eds.): Die Mistel in der Tumortherapie. Grundlagenforschung und Klinik, 55-64, KVC Verlag, Essen]. Diese Hohlbläschen kann man als genuine Liposomen bezeichnen. Die Ausbeute derartiger Liposomen ist relativ gering.

Deshalb wurde gemäß der EP 0310984 B1 versucht, die Liposomen synthetisch gezielt herzustellen. Hierzu wird in einer rotierenden Scheibenvorrichtung ein mehrschichtiger Aufbau aus übereinander aufgespannten Monoschichten, die zusammen damit eine Bischicht bilden, erzeugt, wobei sich die Schichten durch die Zentrifugalkräfte aufgrund der Rotation ausbilden. Durch gezieltes Aufbringen von genuinen Liposomen oder natürlichen Zellen auf diese so erzeugte Bischicht unter gleichzeitigem Einfluss der Zentrifugalkräfte führt dann dazu, dass sich die gebildete Membran aus der Bischicht deformiert und lokal ausbeult, woraufhin bei weiterer Deformation aufgrund der Zentrifugalkräfte eine Abschnürung und ein Einkapseln des genuinen Liposoms bzw. der natürlichen Zellen und damit auch des einzukapselnden Wirkstoffes erreicht werden kann. Der pharmazeutische Wirkstoff kann damit in Form einer liposomalen Einkapselung erzeugt werden. Problematisch an diesem Verfahren ist die gleichmäßige Gewährleistung der komplizierten Strömungsverhältnisse sowie die relativ geringe Ausbeute an liposomal eingekapseltem Wirkstoff.

Die Abschnürung von Liposomen kann auch durch Druck nach dem Vorbild der Extrusion von Roh-Liposomen durch geeignete Membranen (siehe z.B. [Lasch, J., Weissig, V. & Brandl, M. (2003): Preparation of liposomes, in: V.P. Torchillin & V. Weissig (Eds) Liposomes, Practical Approach, Vol. 264, 3-30, Oxford University Press, Oxford] erfolgen. Der Aufwand zur Durchführung der dort genannten Verfahren ist beträchtlich, wohingegen die erzeugten Mengen an Liposomen verhältnismäßig niedrig sind. Auch sind die benötigten Drücke hoch, wodurch ggf. sogar Schädigungen der hergestellten Liposomen auftreten können.

Aufgabe der vorliegenden Erfindung ist es daher, zwei monomolekulare Schichten unterschiedlicher Zusammensetzung zu einer Bischicht zusammenzuführen und durch gezielte Manipulation dieser Bischicht eine Einkapselung der in Form der herzustellenden Liposomen umhüllten Stoffe zu erreichen.

Die Lösung der erfindungsgemäßen Aufgabe ergibt sich aus den kennzeichnenden Merkmalen des Anspruches 1 in Zusammenwirken mit den Merkmalen des Oberbegriffes. Weitere vorteilhafte Ausgestaltungen der Erfindung ergeben sich aus den Unteransprüchen.

Die Erfindung geht aus von einem Verfahren zur Einkapselung von Stoffen in Liposomen, insbesondere zur Wirkstoffeinkapselung in Liposomen einer aus monomolekularen Schichten gebildeten Bischicht. Ein derartiges Verfahren wird dadurch weiterentwickelt, dass in einem ersten Schritt zwei unterschiedliche Monoschichten auf die Oberfläche einer porösen Trägermembran aufgebracht werden, wobei die beiden Monoschichten aufgrund physikalischer Wechselwirkungen zwischen den Substanzen der Monoschichten eine Bischicht mit frei einstellbarer Komposition der inneren und äußeren Seite bilden. Danach werden auf den jeweiligen Außenseiten der die Bischicht bildenden Monoschichten unterschiedliche Fluidvolumina in Form vorzugsweise dünner Filme aufgebracht, die nach der Herstellung der Liposomen die Außenseite der hergestellten Liposomen umgeben und/oder in das Innere der Liposome eingelagert werden. Anschließend wird eine kompressible Membran derart mit der Schichtenanordnung aus Bischicht, Fluidvolumina und poröser Trägermembran in Verbindung gebracht, dass in der kompressiblen Membran angeordnete Poren oder Kanäle, gefüllt mit einem Puffermedium, angrenzend an das in das Innere der Liposomen einzulagernde Fluidvolumen zu liegen kommen. Danach wird die Bischicht im Bereich der Poren der porösen Trägermembran durch einen im Puffermedium einzustellenden Überdruck lokal derart deformiert, daß die Bischicht in die Poren der porösen Trägermembran hineingedrückt wird und sich, das in das Innere der Liposomen einzulagernde Fluidvolumen mindestens teilweise mit sich reißend, einschnürt und von der Bischicht abreißt, woraufhin der abgerissene Bereich der Bischicht sich aufgrund der Minimierung der Grenzflächenenergie zu einem Liposom zusammenschnürt und dabei das mitgerissene Fluidvolumen der in das Innere der Liposomen einzulagernden Substanz einhüllt, sodass das Liposom insbesondere auch durch den Überdruck im Puffermedium durch die poröse Trägermembran hindurch tritt bzw. hindurchgespült werden kann.

Ein derartiges Herstellverfahren bietet eine neue Möglichkeit, technisch relativ einfach und kostengünstig Liposomen herzustellen, die insbesondere für die therapeutische Nutzung geeignet sind, darüber hinaus aber für alle bekannten Nutzungen von Liposomen beispielsweise im Bereich der Nahrungsmitteltechnik oder auch der Kosmetik gleichermaßen geeignet sind. Das Aufbringen der unterschiedlichen Monoschichten auf die Oberfläche der porösen Trägermembran erlaubt es, diese beiden Monoschichten durch physikalische Wechselwirkungen wie etwa die van der Waals'schen Kräfte zwischen zum Beispiel Fettsäureketten zu einer stabilen Bischicht mit unterschiedlichen Eigenschaften der späteren inneren bzw. äußeren Seite eines Liposoms zusammen zu fügen, wobei sich die Substanzkomposition der inneren und äußeren monomolekularen Schicht der Bischicht frei einstellen läßt, was neue Möglichkeiten für die Funktionalität von Liposomenpräparationen eröffnet. Zusätzlich ermöglicht der Herstellungsprozeß eine hohe Einkapselungseffizienz, da das Aufbringen unterschiedlicher Fluidvolumina idealerweise in Form dünner Filme auf die beiden Seiten der Bischicht eine genauere Steuerung der Substanzmengen erlaubt, die zur Einkapselung innerhalb der Liposomen gedacht sind bzw. nach der Herstellung eines Liposoms dieses umgeben, sodass insbesondere bei der Einkapselung von Wirkstoffen in ein Liposom nur geringe Verluste des Wirkstoffes in den Umgebungsbereich des Liposom nach dessen Herstellung auftreten und daher eine hohe Nutzungsrate der üblicherweise teuren Wirkstoffe erzielt werden kann. Die Verwendung der kompressiblen Membran, in die das Puffermedium in den Poren beziehungsweise Kanälen eingelagert ist und bei Kompression der Membran wie bei einem Schwamm freigesetzt wird, erlaubt es, bei Zuordnung der kompressiblen Membranen zu den Flüssigkeitsvolumina und der Bischicht die Bischicht sehr punktgenau im Bereich der Poren beziehungsweise Kanäle der porösen Trägermembran zu manipulieren, wobei bei insbesondere schlagartigem Aufbringen eines Drucks auf die kompressible Membran das nicht oder weitaus weniger kompressible Trägermedium aus den Poren beziehungsweise Kanälen der kompressiblen Membran ebenfalls schlagartig austritt und die angrenzend angeordnete Bischicht im Bereich der Poren beziehungsweise Kanäle der porösen Trägermembran verformt, bis diese aufreißt und einen Teil des Flüssigkeitsvolumens zum Beispiel eines Wirkstoffes mitreißend in die Poren beziehungsweise Kanäle der porösen Trägermembran eintritt und sich danach einschnürt und von der Bischicht abreißt. Dieser abgerissene Bereich der Bischicht wird sich dann zwangsläufig wegen des Bestrebens, einen Zustand minimaler Grenzflächenenergie zur Umgebung einzunehmen, zu einem üblicherweise kugelförmigen Liposomen zusammen schnüren und damit das mitgerissene Fluidvolumen beispielsweise einer Wirksubstanz vollständig umhüllend in das Innere der aus den mitgerissenen Bestandteilen der Bischicht gebildeten Hülle des Liposomens einlagern. Ein derart gebildetes Liposom kann dann ebenfalls durch den Überdruck im Puffermedium, ggf. unterstützt durch eine entsprechende Absaugung, aus dem Kanal bzw. den Poren der porösen Trägermembran ausgespült und abgefiltert oder auf sonstige Weise von der Umgebungsflüssigkeit getrennt werden. Bei diesem Verfahren sind relativ geringe mechanische Belastungen der Wirksubstanzen bzw. Liposomen erzielbar, die beispielsweise bei Hochdruckextrusion als einem anderen bekannten Herstellungsverfahren unvermeidbar sind. Auch kann durch die gezielte Auswahl der porösen Trägermembran und deren Porosität dafür gesorgt werden, daß Liposomen definierter Größe und bei einem hohen Flächenanteil der Porosität an der Oberfläche der porösen Trägermembran auch in verhältnismäßig großer Menge entstehen.

Hierbei ist insbesondere der Auftrag dünner Flüssigkeitsfilme und Monoschichten auf zylindrische Walzenoberflächen von Wichtigkeit, um eine gezielte Synthese zweier Monoschichten zu einer Bischicht sowie das Anlagern von Wirkstoffen bzw. Schutzschichten und Rezeptoren an die Innen- bzw. Außenseite der Bischicht zu bewerkstelligen. Eine einfache Variation der Verfahrensparameter läßt sich durch die Beeinflussung des druckgesteuerten Zerreißens der Bischichten und den Transport der sich bildenden Liposomen durch uniforme Poren erzielen, wobei zur Effektivität des Verfahrens auch das Absaugen der Liposomensuspension hinter der porösen Trägermembran in einem Drainage-System mit möglichst geringem Totvolumen beiträgt.

Für die Gleichmäßigkeit der Bildung der Liposomen ist es von Vorteil, wenn die poröse Trägermembran Poren mit exakter Porengröße aufweist, wofür beispielsweise auch eine Filtermembran mit höherer Porosität als poröse Trägermembran verwendet werden kann. Derartige poröse Trägermembranen, die beispielsweise mit Hilfe nanotechnologischer Verfahren, vorzugsweise mit Hilfe der sogenannten track-etch-Technik hergestellt werden können, weisen sehr gleichmäßige und bezogen auf den Flächenanteil der Trägermembran sehr viele Poren beziehungsweise Kanäle auf, durch die die vorstehend beschriebenen Liposomenbildung ablaufen kann.

Von besonderem Vorteil ist es, wenn die poröse Trägermembran aus einer Polycarbonat-Membran (PC-Membran) gebildet wird, bei der die Porosität als durchgängige Poren oder Kanäle ausgebildet ist. Hierdurch werden die oberseitig der Trägermembran im Bereich des Eintritts in die Poren beziehungsweise Kanäle gebildeten Liposomen nach dem Aufreißen der Bischicht durch den weiter wirkenden Druck für das Aufreißen der Bischicht durch die ganze Pore beziehungsweise den ganzen Kanal der porösen Trägermembran hindurch befördert und treten an der anderen Seite wieder aus der porösen Trägermembran aus. Selbstverständlich ist auch denkbar, dass die poröse Trägermembran aus einem anderen Material mit durchgängigen Poren und hoher Porosität bestehen kann.

Eine besonders hohe Ausbeute an Liposomen lässt sich dann erreichen, wenn die poröse Trägermembran eine Porosität mit hoher Porendichte, vorzugsweise bis 25% der Oberfläche der Trägermembran aufweist. In weiterer Ausgestaltung kann vorgesehen werden, daß die durchgängigen Poren oder Kanäle einen Durchmesser von 10 nm bis zu 20 µm aufweisen, wodurch die Größe der hergestellten Liposomen im selben Größenbereich liegen kann.

Weiterhin trägt zur Effektivität des Verfahrens bei, dass die Bischicht aufgrund der hohen Porendichte der porösen Trägermembran nahezu vollständig in kugelförmige Liposomen umgewandelt wird. Hierdurch wird nur wenig Substanz der Bischicht nicht in Oberflächen von Liposomen umgewandelt und damit geht nur wenig Substanz der aufgespannten Bischicht verloren.

Von besonderem Vorteil hinsichtlich der späteren Verwendung der Liposomen ist es, wenn die in den Liposomen innen liegende Oberflächenschicht der Bischicht derart ausgebildet wird, dass die in den Liposomen einzuhüllenden Stoffe an dieser Innenschicht adsorbieren oder in diese eingebettet werden. Hierdurch lässt sich erreichen, dass das Fluidvolumen, das an dieser Seite der Bischicht angelagert wird, besonders affin zu dieser Seite der Bischicht ist und beim Abreißen der Bischicht sehr viel dieses Fluidvolumens mitgerissen wird. Ebenfalls ist es in anderer Ausgestaltung denkbar, dass die in den Liposomen außen liegende Monoschicht der Bischicht derart ausgebildet wird, dass die die Liposomen umhüllenden Stoffe an dieser Außenschicht adsorbieren, wobei in einer Weiterbildung die die Liposomen umhüllenden Stoffe an dieser Außenschicht als sterische Schutzschichten oder Rezeptoren aufgebracht werden können. Hierdurch lässt sich insbesondere auch die Außenseite der Liposomen gezielt an die geforderten Eigenschaften beispielsweise einer therapeutischen Anwendung der Liposomen anpassen, in dem entsprechende die Außenschicht der Liposomen umhüllende Stoffe besonders gut an dieser Fläche der Bischicht angelagert werden können beziehungsweise fest an dieser anhaften.

Eine genaue Steuerung der Schichtdicken bzw. auch der zu umhüllenden Volumina lässt sich dann erreichen, wenn die Fluidvolumina in Form sehr dünner Fluidfilme auf die Bischicht aufgebracht bzw. an der Bischicht angelagert werden, insbesondere wenn die Fluidvolumina Schichtdicken bis zu 1 µm aufweisen. Hierdurch ist die Verwendung dieser Fluidvolumina sehr effektiv und es geht wenig dieser Fluidvolumina verloren, da diese Fluidvolumina weitgehend beim Bilden der Liposomen aufgebraucht werden.

Eine erste denkbare Ausgestaltung des Verfahrens sieht vor, das Verfahren kontinuierlich mittels einer Anordnung sich aufeinander abwälzender Walzen ausgeführt wird, auf denen die verschiedenen Membranen und Flüssigkeitsvolumina aufgespannt und durch Abwälzen der verschiedenen Walzen miteinander in Kontakt gebracht werden. Durch ein derartiges kontinuierliches Verfahren können bei sehr gleichbleibender Qualität auch größere Mengen von Liposomen hergestellt werden, wobei durch die kontinuierliche Arbeitsweise einer entsprechenden Vorrichtung eine gute Steuerung des Verfahrens erreicht werden kann.

Für kleinere herzustellende Mengen von Liposomen oder auch für das Verfahren kann es von Vorteil sein, wenn das Verfahren diskontinuierlich mittels einer Anordnung von Walzen und einem planaren Träger ausgeführt wird, auf dem die verschiedenen Membranen und Flüssigkeitsvolumina aufgespannt und/oder durch Abwälzen der Walzen miteinander in Kontakt gebracht werden. Hierbei können Verfahrensparameter einfach geändert beziehungsweise das Aufspannen entsprechender Schichten beziehungsweise Filme auf einem planaren Träger einfacher realisiert werden.

Die Erfindung betrifft weiterhin eine diskontinuierlich arbeitende Vorrichtung zur Einkapselung von Stoffen in Liposomen, insbesondere zur Durchführung eines Verfahrens gemäß Anspruch 1. Bei einer derartigen Vorrichtung weist die Vorrichtung einen planaren Träger auf, auf dem eine poröse Trägermembran aufgespannt ist, wobei mindestens eine Walze nacheinander die verschiedenen Membranen und Fluidvolumina, die auf der mindestens einen Walze vorab auftragbar sind, durch eine Relativbewegung auf der porösen Trägermembran abwälzend mit der porösen Trägermembran in Kontakt bringt. Hierdurch ist ähnlich wie beim altertümlichen Hochdruck mittels einer Handpresse eine gezielte Beeinflussung der Oberfläche der porösen Trägermembran durch die Walze bzw. auf der Walze aufgebrachte Fluidvolumina oder Schichten erzielbar, sodass die Prozeßführung eines Verfahrens gemäß Anspruch 1 sehr überschaubar und hinsichtlich der Verfahrensparameter leicht veränderbar beeinflusst werden kann.

Von Vorteil für die Rückgewinnung der Liposomen aus den in der Vorrichtung verarbeiteten Fluide ist es, wenn der planare Träger eine Platte mit Drainagerillen aufweist. Durch diese Drainagerillen kann das aus der porösen Trägermembran einseitig austretenden Fluidvolumen einfach abgeführt und zur Trennung der gewonnenen Liposomen weiterverarbeitet werden. Hierbei kann in weiterer Ausgestaltung der planare Träger eine dünne Sinterplatte, vorzugsweise aus Polyethylen oder Edelstahl aufweisen, die zur Stabilisierung der üblicherweise sehr dünnen porösen Trägermembran dient, die gemeinsamen mit der Sinterplatte auf der Platte mit den Drainagerillen aufgebracht werden kann.

Eine weitere Ausgestaltung der Vorrichtung sieht vor, dass eine kompressible Membran auf der mindestens einen Walze aufgebracht ist und mit einstellbarem Druck relativ zu dem planaren Träger verschiebbar und dabei auf dem planaren Träger abwälzbar ist. Dieses Verfahren entspricht grundsätzlich dem bekannten altertümlichen Hochdruck mittels einer abrollenden Handpresse und sorgt für eine sehr gleichmäßige Belastung des planaren Trägers beim Abrollen der Walze, wodurch die Bedingungen zur Entstehung der Liposomen sehr gezielt geändert werden können.

Selbstverständlich ist es auch denkbar, statt nur einer Walze für jeden der Beschichtungs- und Bearbeitungsvorgänge eine separate Übertragungswalze vorzusehen, die nacheinander in die Vorrichtung einbringbar und mit der porösen Trägermembran oder der Walze in Kontakt zu bringen sind. Hierdurch kann eine getrennte Vorbereitung der einzelnen Schritte der Beschichtung- und Bearbeitungsvorgänge in Form der einzelnen Übertragungswalzen vorgenommen werden, sodass der eigentliche Vorgang des Bildens der Liposomen relativ schnell und auch mit hoher Taktfrequenz ausgeführt werden kann.

Wichtig ist es hierbei, daß die Relativbewegung zwischen dem planaren Träger und der mindestens einen Walze schlupffrei erfolgt, sodass ein immer gleichmäßiger Auftrag der einzelnen Schichten auf der porösen Trägermembran erfolgt.

Von Vorteil ist es, wenn die die Monoschichten bildenden Substanzen als Lösung in einem Lösungsmittel, insbesondere Ethanol, auf die Übertragungswalze übertragbar sind. Derartige Verwendungen von Lösungsmitteln zur Herstellung von Monoschichten sind grundsätzlich gut bekannt und daher gut beherrschbar, sodass sich definierte Schichtdicken und Eigenschaften der Schichten einfach und reproduzierbar herstellen lassen. Hierbei ist es weiterhin denkbar, dass die die Monoschichten bildenden Substanzen nach dem Übertragen auf die Übertragungswalze auf der Übertragungswalze trocknen und als getrocknete Filme auf die auf die Membranen aufgetragenen Fluidvolumina übertragbar sind. Hierbei ist es zur Ablösung der aufgetragenen Filme von Vorteil, wenn die auf die Membranen aufgetragenen Fluidvolumina die getrockneten Filme wieder anlösen und auf den Fluidvolumina als Monoschichten ablagern.

Eine einfache Beeinflussung der Vorgänge beim Entstehen der Liposomen lässt sich durch Variation der Elastizität der Beschichtung der Walze mit der unter der kompressiblen Membran liegenden Elastomerschicht und des Anpressdrucks zwischen dieser Walze und dem planaren Träger erreichen. Hierdurch lässt sich die Druckverteilung in den Poren des porösen Trägermaterials bei der Herstellung der Liposomen in gewünschter Weise einstellen. Für die Extrusion der Bischicht durch die poröse Trägermembran und das Bilden der Liposomen ist ein bestimmter Mindestdruck notwendig, der von mehreren Faktoren abhängt (Fluidität der Bischicht, Porengröße etc.).

Eine Vermeidung unnötiger Totvolumina bei der Rückgewinnung der Liposomen aus dem Fluidvolumen, das aus der porösen Trägermembran auf der anderen Seite wieder austritt, lässt sich erreichen, wenn die Drainage der hergestellten Liposomen durch eine lokale Absaugung nur desjenigen Bereichs des planaren Trägers erfolgt, in dem die Walze und das poröse Trägermaterial beim Abwälzen der Walze jeweils aufeinander gepresst werden. Dies lässt sich beispielsweise dadurch erreichen, dass zur Drainage nur derjenige Abschnitt des planaren Trägers mit einer feststehenden Absaugung in wirkende Verbindung gebracht wird, in dem die Walze und das poröse Trägermaterial beim Abwälzen der Walze jeweils aufeinander gepresst werden. Hierdurch wird nur sehr wenig des jeweiligen Fluidvolumens abgesaugt, sodass auch die Rückgewinnung der hergestellten Liposomen besonders einfach möglich ist.

Die Erfindung betrifft weiterhin eine kontinuierlich arbeitende Vorrichtung zur Einkapselung von Stoffen in Liposomen, insbesondere zur Durchführung eines Verfahrens gemäß Anspruch 1, bei der die Vorrichtung einen walzenförmigen Träger aufweist, auf dem eine poröse Trägermembran aufgespannt ist und eine Anordnung sich aufeinander abwälzender Walzen die verschiedenen Membranen und Fluidvolumina durch rotatorische Relativbewegungen auf der porösen Trägermembran abwälzend mit der porösen Trägermembran in Kontakt bringt. Eine derartige kontinuierliche Gewinnung von Liposomen durch sich ständig wiederholende rotatorische Relativbewegungen erlaubt eine Herstellung auch größerer Mengen von Liposomen, darüber hinaus sind die Verarbeitungsverhältnisse der Fluidvolumina bzw. der Bischichten sehr gleichmäßig und gut beeinflussbar.

Hierbei ist es von Vorteil, wenn der walzenförmige Träger mit der porösen Trägermembran auf einem ebenfalls walzenförmigen Träger mit einer kompressiblen Membran abwälzt, wobei vorteilhaft die Beschichtung der sich aufeinander abwälzenden Walzen mit den verschiedenen Fluidvolumina gleichzeitig aufgrund der räumlichen Zuordnung der Walzen zueinander erfolgt und die zeitliche Abstimmung der Auftragsprozesse auf die Walzen sowie der notwendigen Trocknungsprozesse von Schichten auf den Walzen durch Variation der Walzendurchmesser bei einheitlicher Umfangsgeschwindigkeit vorgenommen werden kann.

Die Beschichtung der Walze mit den verschiedenen Fluidvolumina kann bei der kontinuierlichen sowie bei der diskontinuierlichen Vorrichtung in grundsätzlich bekannter Weise durch eine Anordnung aus Schöpfwalzen, Rakeln und Übertragungswalzen durchgeführt werden, mit der die Schichtdicken der aufgetragenen Fluidvolumina gezielt eingestellt werden. Die Rakeln können an allen Walzen an beliebiger Position angebracht sein und unterschiedliche Geometrien aufweisen, wie z.B. zylindrische Rundstäbe, Streichmesser, Gummilippen usw. Sie können statisch angebracht oder gleich- oder gegensinnig drehend wirken. Auch ist es denkbar, daß die Schichtdicken der aufgetragenen Fluidvolumina durch Verdunstung beeinflusst wird.

Eine besonders bevorzugte Ausführungsform des erfindungsgemäßen Verfahrens sowie der erfindungsgemäßen Vorrichtungen zeigt die Zeichnung.

Es zeigen:
- Figur 1 -: ein Ablaufschema des erfindungsgemäßen Verfahrens zur effizien- ten Wirkstoffeinkapselung in Liposomen mit frei einstellbarer Kom- position der inneren und äußeren Seite der Bischicht in Form eines schematischen Stadienplans,
- Figur 2 -: eine sehr schematische Darstellung einer kontinuierlich arbeitenden Vorrichtung zur Durchführung des Verfahrens gemäß Figur 1,
- Figur 3 -: eine konstruktive Durchgestaltung einer diskontinuierlich arbeitenden Vorrichtung zur Durchführung des Verfahrens gemäß Figur 1 in einer räumlichen Gesamtansicht sowie Darstellung einiger Einzelteile,
- Figur 4 -: Darstellungen von weiteren Ansichten der Vorrichtung gemäß Figur 3,
- Figur 5 -: eine Seitenansicht der Vorrichtung gemäß Figur 3,
- Figur 6 -: eine Draufsicht der Vorrichtung gemäß Figur 3.

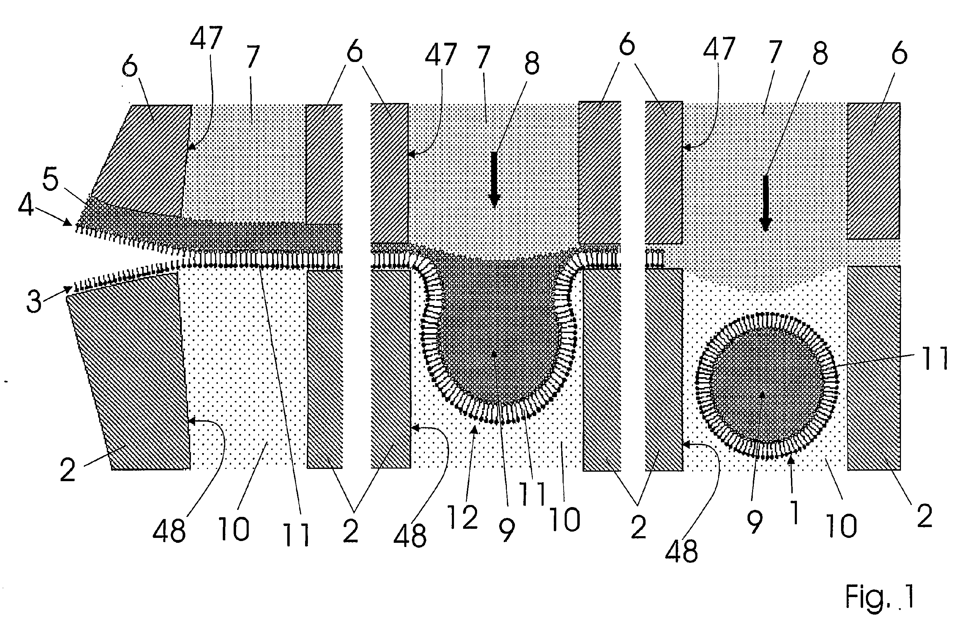

Der Ablauf eines Verfahrens gemäß Anspruch 1 wird in starker Vereinfachung, gleichwohl aber in seinen Grundsätzen eindeutig in der Figur 1 dargestellt, in der ein stark vergrößerter Ausschnitt der schichtweisen Anordnung der einzelnen Schichten und Membranen bei der Herstellung von Liposomen gemäß dem erfindungsgemäßen Verfahren zu erkennen ist.

Im linken Teil der Figur 1 werden in einem ersten Schritt zwei unterschiedliche Monoschichten 3, 4 auf die Oberflächen zweier poröser Trägermembranen 2, 6 aufgebracht, wobei die beiden Monoschichten 3, 4 aufgrund physikalischer Wechselwirkungen wie etwa van-der-Waalsscher Kräfte zwischen den Substanzen der Monoschichten 3, 4 eine Bischicht 11 mit frei einstellbarer Komposition der inneren und äußeren Seite bilden. In der Figur 1 nicht detaillierter dargestellt sind in einem vorgelagerten Verfahrensschritt auf den jeweiligen Außenseiten der die Bischicht 11 bildenden Monoschichten 3, 4 unterschiedliche Fluidvolumina 5, 10 in Form vorzugsweise dünner Filme aufgebracht, die nach der Herstellung der Liposomen 1 die Außenseite der hergestellten Liposomen 1 umgeben und/oder in das Innere der Liposomen 1 eingelagert werden. In Fluidvolumen 5 ist der Wirkstoff 9 gelöst, der im Inneren der Liposomen 1 eingelagert werden soll. Die Lösung 10 befindet sich nach der Bildung der Liposomen 1 im Liposomenaußenraum.

Die poröse Trägermembran 2 weist hierbei Poren bzw. durchgehende Kanäle 48 definierten Durchmessers auf, die mit einem hohen Flächenanteil an der gesamten Oberfläche der porösen Membran 2 vorgesehen sind. Derartige Poren bzw. Kanäle 48 werden beispielsweise mit Hilfe der Nanotechnologie hergestellt und entsprechende Membranen 2 sind marktgängig lieferbar. Die aus den beiden Monoschichten 3, 4 gebildete Bischicht 11 überspannt hierbei die Poren bzw. Kanäle 48, wobei die Bischicht 11 durch das Fluidvolumen 10 aus der Lösung für den Liposomenaußenraum sowie die Pufferlösung 7 umgeben ist, die in Poren bzw. Kanälen 47 einer kompressiblen. Membran 6 aufgenommen ist. Diese kompressible Membran 6 wird beispielsweise durch eine Rotation einer hier nicht weiter dargestellten Trägerwälze oder dgl. im Verlauf des Verfahrens derart mit der Schichtenanordnung aus Bischicht 11, Fluidvolumina 5, 10 und poröser Trägermembran 2 in Verbindung gebracht, dass das Fluidvolumen 5 in das Liposom 1 mit dem Wirkstoff 9 eingebracht wird. Die Poren oder Kanäle 47 der kompressiblen Membran 6 und die Poren oder Kanäle 48 der porösen Membran 2 überlappen einander zumindest teilweise, wobei durch die hohen erzielbaren Porendichten handelsüblicher Membranen ein nennenswerter Teil der Poren oder Kanäle 47 mit entsprechenden Poren oder Kanälen 48 auch ohne genaue Zuordnung der Membranen 2, 6 zueinander in fluidleitende Verbindung gebracht werden können.

Im mittleren Teil der Figur 1 wird die Bischicht 11 im Bereich der Poren bzw. Kanäle 48 der porösen Trägermembran 2 durch einen im Puffermedium 7 einzustellenden Überdruck 8 lokal derart deformiert, dass die Bischicht 11 abschnittsweise in die Poren bzw. Kanäle 23 der porösen Trägermembran 2 hineingedrückt wird und sich das in das Innere der Liposomen 1 einzulagernde Fluidvolumen 9 zumindest teilweise mit sich reißend, einschnürt und von der Bischicht 11 abreißt, woraufhin der abgerissene Bereich 12 der Bischicht 11 sich aufgrund der Minimierung der Grenzflächenenergie zu einem Liposom 1 zusammenschnürt und dabei das mitgerissene Fluidvolumen 5 der in das Innere der Liposomen 1 einzulagernden Substanz 9 einkapselt, sodass das Liposom 1 durch den Überdruck 8 im Puffermedium 7 durch die poröse Trägermembran 2 hindurchgespült werden kann.

Der Aufbau des Drucks 8 innerhalb der Pufferlösung 7 kann z.B. durch mechanischen Druck auf die kompressible Membran 6 hervorgerufen werden, etwa indem eine hier nicht weiter dargestellte Druckwalze über die kompressible Membran 6 abrollt und dabei lokal eine Kompression der kompressiblen Membran 6 hervorruft. Da die Pufferlösung 7 inkompressibel ist, wird die Pufferlösung in Richtung auf die Poren 48 der porösen Trägermembran 2 hin ausweichen und dabei die Bischicht 11 ausstülpen. In Abhängigkeit vom Maß der Deformation der kompressiblen Membran 6 bzw. der Geschwindigkeit der Deformation wird das Fluidvolumen 5 des Wirkstoffes 9, das möglicherweise durch Adhäsionsvorgänge an der Bischicht 11 besonders gut anhaftet, mit der Bischicht 11 ebenfalls in Richtung auf die Poren 48 der porösen Trägermembran 2 beschleunigt und bildet die Ausstülpung 12 mit dem darin teilweise eingehüllten Wirkstoffvolumen 9. Bleibt der Druck 8 aufrechterhalten oder wird er noch weiter erhöht, so reißt der schon ausgestülpte Bereich 12 der Bischicht 11 endgültig von der Bischicht 11 ab und wird sich zur Minimierung der Grenzflächenspannung, wie im rechten Teil der Fig. 1 erkennbar, zu einem im wesentlichen kugelförmigen Liposom 1 verformen, das den von dem Fluidvolumen 5 mitgerissenen Wirkstoff 9 vollständig umhüllt.

Die Phasengrenzen zwischen dem Fluidvolumen 5 des Wirkstoffes 9 und der Pufferlösung 7 sowie der Lösung 10 sind hier schematisch dargestellt. Da alle drei Flüssigkeiten wässrig und damit mischbar sind, wird es sowohl zu einer konvektiven als auch diffusiven Durchmischung kommen. Eine effiziente Einkapselung von Wirkstoffen 9 aus dem Fluidvolumen 5 des Wirkstoffes in den Innenraum der Liposomen 1 ist dennoch möglich, wenn die Innenseite der Bischicht 11 (Monoschicht 4) so aufgebaut wird, dass die Wirkstoffe an ihr adsorbieren. Dasselbe gilt für die Stoffe (z.B. für die sterische Schutzschicht oder Rezeptoren), die aus der Lösung 10 an die Außenseite der Liposomen 1 (Monoschicht 3) binden sollen. Somit erweist sich die gezielt einstellbare Zusammensetzung der beiden Monoschichten 3, 4 als Voraussetzung sowohl für eine hohe Einkapselungseffizienz als auch für den geordneten Strukturaufbau der Liposomenschale.

Dieses in der Figur 1 sehr schematisch dargestellte Verfahren kann nun auf unterschiedliche Weise vorrichtungsmäßig umgesetzt werden.

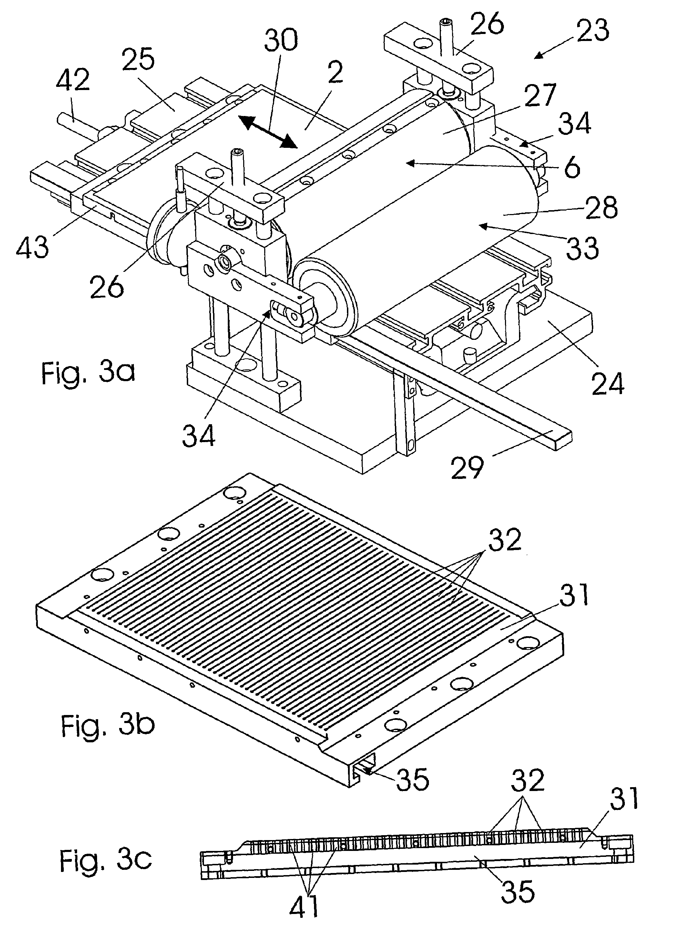

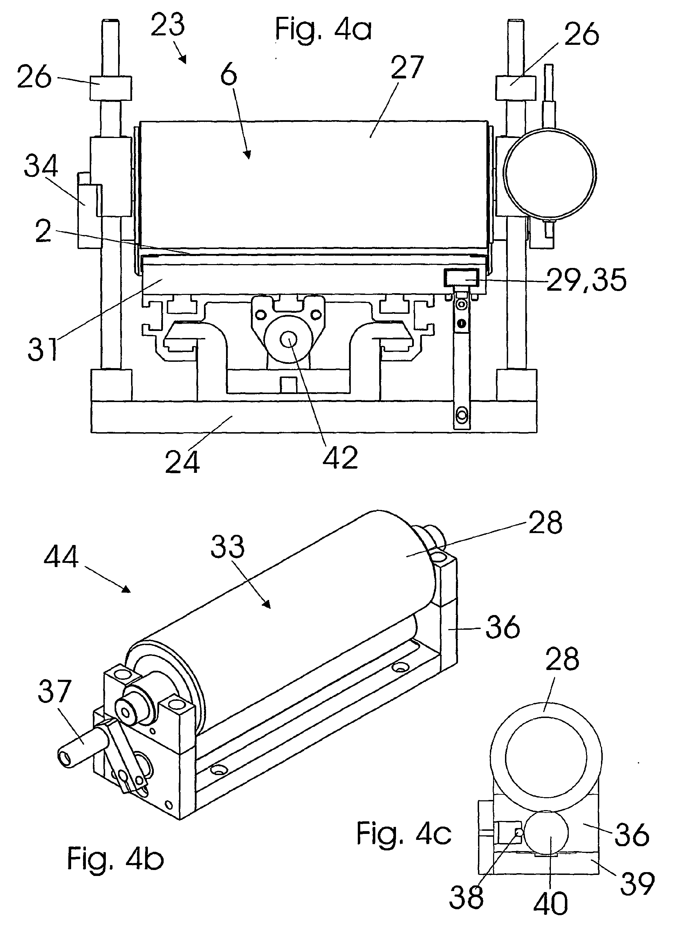

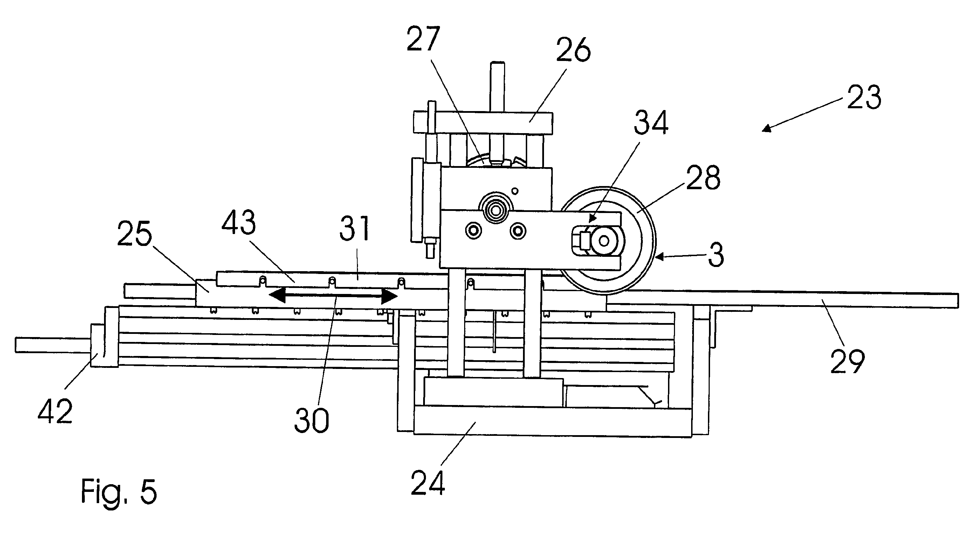

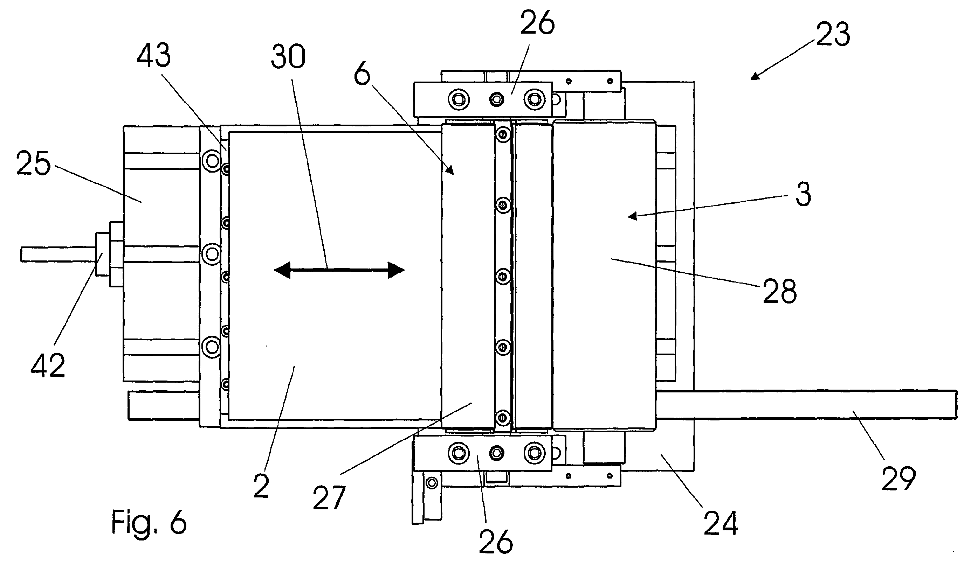

Die Figuren 3 bis 6 beziehen sich auf eine diskontinuierlich arbeitende Vorrichtung 23 für ein Batch-Verfahren, die nur in ihren Grundzügen und nicht hinsichtlich aller konstruktiven Details beschrieben werden soll, soweit diese Details nicht zum Verständnis des Verfahrens bzw. der Funktion der Vorrichtung 23 von wesentlicher Bedeutung sind.

Das Kernstück der Vorrichtung 23 für das Batch-Verfahren ist eine Nutenplatte 31 mit feinen Drainage-Rillen 32. Auf diese Nutenplatte 31 wird eine dünne, nicht weiter dargestellte Sinterplatte (entweder aus Polyethylen etwa mit einer Dicke von 0,6 mm oder aus Edelstahl mit einer Dicke von 1,5 mm) aufgespannt und darauf die poröse Membran 2 mit einer Dicke von ca. 20 µm mit durchgängigen Poren bzw. Kanälen 23. Beide Folien, poröse Membran 2 und darunterliegende Sinterplatte, werden an den Rändern der Nutenplatte 31 mit umlaufenden Klemmleisten 43 festgeklemmt.

Die kompressible Membran 6 wird auf eine Walze 27 gespannt. Diese Membran 6 soll kompressibel sein, sodass sie unter Druck schwammartig Flüssigkeit abgibt. Die membranbespannte Walze 27 wird in ein Trägergestell 26 fest eingespannt. Durch die variable Aufhängung in dem Trägergestell 26 wird diese Walze 27 mit einstellbarem Druck gegen die Nutenplatte 31 gepresst. Die Nutenplatte 31 ist auf einem mittels Antrieb 42 beweglichen Schlitten 25 montiert und kann unter der Walze 27 in beide Richtungen 30 bewegt werden. Dadurch rollen sich die beiden Membranen, nämlich die kompressible Membran 6 und die poröse Trägermembran 2 aufeinander ab und lösen die vorstehend beschriebene Bildung der Liposomen 1 aus.

Die Beschichtung der auf die Walze 27 bzw. Nutenplatte 31 aufgespannten Membranen 2, 6 mit dünnen Flüssigkeitsfilmen 5, 7, 10 bzw. Monoschichten 3, 4 wird wie folgt bewerkstelligt:

In separaten Schöpfstationen 44 mit einem Halter 36 werden die entsprechenden Flüssigkeiten 5, 7, 10, 3, 4 in flachen Wannen 39 bereitgestellt, in die eine hier mit einer Handkurbel 37 antreibbare Schöpfwalze 40 eingetaucht ist, die bei Rotation einen dünnen Film 33 aus einer der Flüssigkeiten 5, 7, 10, 3, 4 mitnimmt. Die Dicke des Films 33 wird durch eine Rakel 38, die gegen die Schöpfwalze 40 gepresst wird, auf ein Minimum reduziert. Die Schöpfwalze 40 rollt gegen die gummibeschichtete Walze 28 ab, mit der dann die Beschichtung mit dem jeweiligen Flüssigkeitsfilm 33 der kompressiblen Membran 6 und der porösen Trägermembran 2 vorgenommen werden kann. Ggf. kann die Dicke des Flüssigkeitsfilms 33 durch Verdunstung (unter Einhaltung der dafür erforderlichen Trocknungszeit) weiter reduziert werden. Sobald der Flüssigkeitsfilm 33 auf der Walze 28 die gewünschte Dicke erreicht hat, wird die Walze 28 in eine zweite Walzenaufhängung 34 der Vorrichtung 23 eingehängt. Diese Walzenaufhängung 34 ist so einstellbar, dass die Walze 28 entweder gegen die Walze 27 mit der kompressiblen Membran 6 oder gegen die poröse Trägermembran 2 auf der Nutenplatte 31 angedrückt werden kann. Für die Auftragung der drei Flüssigkeitsfilme 5, 7, 10 und zwei Monoschichten 3, 4 sind somit fünf Walzen 28 notwendig, die hintereinander in die Walzenaufhängung 34 eingesetzt und einmalig über ihre Umfangsfläche abgerollt werden, so dass sich der Film 33 in der beschriebenen Weise überträgt.

Für die Vorbereitung zum Auftrag der Monoschichten 3, 4 wird dasselbe Prinzip mit Schöpfstationen 44 wie für die Flüssigkeitsfilme 5, 7, 10 angewendet. Die die Monoschichten 3, 4 konstituierenden Substanzen (Lipide) werden in Ethanol gelöst und als Ethanol-Film über die Schöpfwalze 40 auf die Walze 28 aufgetragen. Nach einmaligem Abrollen der gummierten Walze 28 auf der Schöpfwalze 40 wird der Ethanolfilm jedoch vollständig zur Trocknung gebracht. Die trockenen Lipide werden bei Abrollen der Walze 28 auf der kompressiblen Membran 6 bzw. der porösen Trägermembran 2 durch die auf diese Membranen 2, 6 zuvor aufgetragenen Flüssigkeitsfilme 5, 7, 10 gelöst und bilden Monoschichten 3, 4 auf diesen Flüssigkeitsfilmen 5 und 10.

Ein Zyklus des diskontinuierlichen Verfahrens läuft in folgender Reihenfolge ab:
a) Auftragung der Flüssigkeitsfilme 5, 7, 10 über Schöpfwalzen 40 auf Walzen 28;
b) Übertragung der Flüssigkeitsfilme 5, 7, 10 von den Walzen 28 auf die mit der kompressiblen Membran 6 bezogene Walze 27 bzw. auf die mit der porösen Trägermembran 2 bespannte Nutenplatte 31;
c) Übertragen der getrockneten Lipidschichten 3, 4 von Walzen 28 auf die Oberflächen der unter b) genannten dünnen Flüssigkeitsfilme 5, 7, 10;
d) Abrollen der mit der kompressiblen Membran 6 bezogenen Walze 27 auf der mit der porösen Trägermembran 2 bespannten Nutenplatte 31. Die Gummierung der Walze 27 ist austauschbar, sodass durch Variation des Anpressdrucks und der Elastomer-Härte der Gummierung der Walze 27 die notwendige Druckverteilung innerhalb der Poren bzw. Kanäle 23 eingestellt werden kann, mit der Liposomen 1 erzeugt werden können.
e) Die gebildeten Liposomen 1 werden durch die Nutenplatte 31 abgesaugt. Jede der quer eingefrästen Drainagekanäle 32 endet in einer Absaugbohrung 41 durch die Nutenplatte 31. In der Rückseite der Nutenplatte 31 ist eine längs eingefräste Absaugnut 35 vorgesehen. Diese Absaugnut 35 wird ausgefüllt durch eine durchgehende Absaugleiste 29 mit nicht weiter dargestellter Teflon-Abdichtung an ihrer Oberfläche. Die Absaugleiste 29 ist fest mit dem Gestell 24 der Vorrichtung 23 verbunden und damit unbewegt gegenüber der Walze 27, d.h. die Nutenplatte 31 wird relativ zur Walze 27 und zur Absaugleiste 29 bewegt, während die beiden Membranen 2, 6 aneinander abrollen (Schritt d). In der Absaugleiste 29 ist eine Durchgangsbohrung 46 direkt unter der Walze 27, sodass die Liposomenlösung jeweils aus dem Drainagekanal 32, die sich unter der Anpreßfläche von Walze 27 und Nutenplatte 31 befindet, abgesaugt werden kann.

Durch den unter e) beschriebenen Absaugvorgang ist das Totvolumen auf ein Minimum reduziert.

Die Vorrichtung 23 ist so ausgelegt, dass damit kleine Mengen (ca. 1 ml) an Liposomenlösungen erzeugt werden können, die für eine physikalisch-chemische Charakterisierung und Analytik ausreichen.

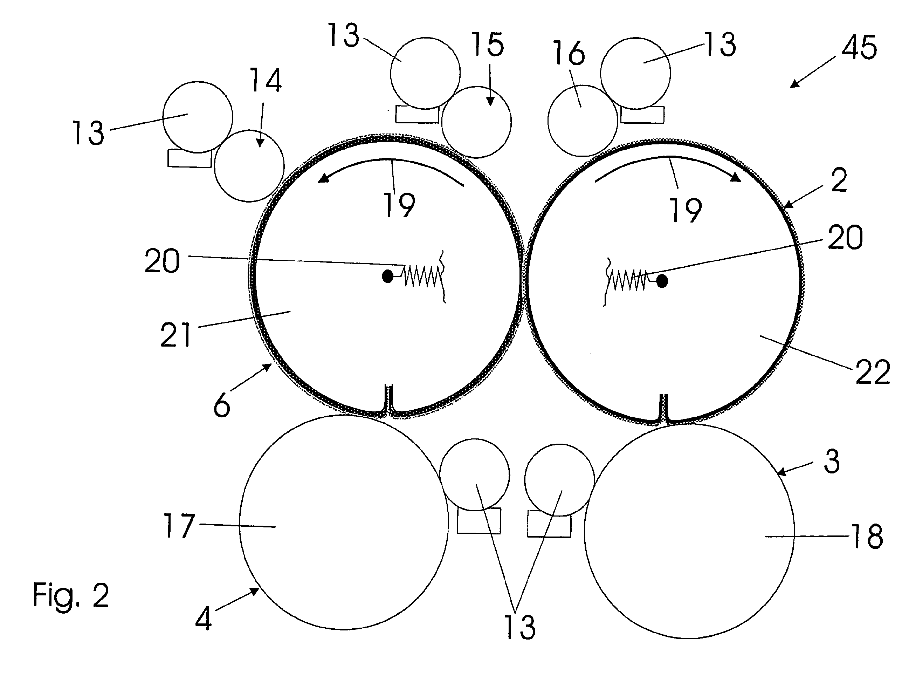

Für größere Mengen (100 ml und mehr) ist eine kontinuierliche Vorrichtung 45 notwendig, die sehr prinzipiell in der Figur 2 dargestellt ist.

Die Vorrichtung 45 weist zwei wesentliche Unterschiede gegenüber der Vorrichtung 23 auf:
1) Die poröse Trägermembran 2 ist hier nicht mehr auf einer planaren Trägerplatte (Nutenplatte 31) eingespannt, sondern auf einer genuteten Walze 22.
2) Die Beschichtung mit Flüssigkeitsfilmen 5, 7, 10 und Monoschichten 3, 4 verläuft nicht mehr getrennt in zeitlich aufeinanderfolgenden Schritten, sondern gleichzeitig in einem räumlich hintereinandergeschalteten Aufbau, wodurch eine kontinuierliche Durchführung des Verfahrens ermöglicht wird.

Die Funktion der Vorrichtung 45 für die kontinuierliche Durchführung des Verfahrens ist aus der Figur 2 zu ersehen, bei der sich alle zu der Vorrichtung 23 schon beschriebenen Abläufe beim kontinuierlichen Verfahren simultan abspielen. Hinsichtlich der einzelnen Vorgänge sei daher ausdrücklich auf die Beschreibung der Vorrichtung 23 verwiesen und hier nur die Unterschiede näher erläutert. Da alle sich berührenden Walzen 14, 15, 16, 17, 18, 21, 22 in gegensinniger Drehrichtung 19 ohne Schlupf und im Falle der Walzen 21 und 22 unter Federvorspannung 20 aneinander abrollen, ist die Umfangsgeschwindigkeit für alle Walzen 14, 15, 16, 17, 18, 21, 22 gleich. Da jedoch die verschiedenen Prozeßschritte a) bis e) gemäß der vorstehenden Beschreibung zur Vorrichtung 23 unterschiedliche Trocknungszeiten etc. erfordern könnten, müssen die Umfangsverhältnisse der jeweiligen Walzen 14, 15, 16, 17, 18, 21, 22 an die gewünschten Winkelgeschwindigkeitsverhältnisse angepaßt werden.

Die Bestimmung der erforderlichen Geschwindigkeitsverhältnisse und der daraus resultierenden Umfangsverhältnisse lässt sich auch aus Erfahrungswerten des Betriebes der Vorrichtung 23 des diskontinuierlichen Verfahrens gewinnen.

Der in Figur 2 skizzierte Prozess lässt sich als kontinuierliches Verfahren aufbauen, indem die beiden Membranen 2, 6 auf zylindrische Walzen 22, 21 aufgespannt werden, die sich in gegensinniger Drehrichtung 19 aneinander abrollen. Der Auftrag der Monoschichten 3, 4 und der dünnen Flüssigkeitsfilme 5, 7, 10 kann durch zusätzliche Walzen 14, 15, 16, 17, 18 in schon vorstehend grundsätzlich beschriebenen Schöpfstationen 13 erfolgen, die in der Reihenfolge der Beschichtung an den auf den Walzen 21, 22 aufgespannten Membranen 2, 6 abrollen. Für den Auftrag der Monoschichten 3, 4 werden die dafür z.B. verwendeten Lipidmischungen zunächst in ethanolischer Lösung in Schöpfstationen 13 auf eine Walze 17, 18 aufgetragen. Nach Verdunsten des Ethanolfilms wird die trockene Lipidschicht bei nur leichter Berührung auf den mit den Flüssigkeitsfilmen 5, 7, 10 überzogenen Walzen 21, 22 mit den Membranen 2, 6 abgerollt, um dadurch die Monoschichten 3, 4 zu übertragen.

### Sachnummernliste

- 1 -: Liposom
- 2 -: poröse Trägermembran
- 3 -: Monoschicht für Außenseite des Liposoms
- 4 -: Monoschicht für Innenseite des Liposoms
- 5 -: einzukapselnde Wirkstofflösung
- 6 -: kompressible Membran
- 7 -: Pufferlösung
- 8 -: Druckaufbau
- 9 -: eingekapseltes Wirkstoffvolumen
- 10 -: Lösung für Liposomenaußenraum
- 11 -: Bischicht
- 12 -: Ausstülpung der Bischicht
- 13 -: Schöpfstation
- 14 -: Walze Wirkstofflösung
- 15 -: Walze Pufferlösung
- 16 -: Walze Lösung Liposomenaußenraum
- 17 -: Walze Monoschicht
- 18 -: Walze Monoschicht
- 19 -: Drehrichtung
- 20 -: Federvorspannung
- 21 -: Walze
- 22 -: Walze
- 23 -: diskontinuierliche Vorrichtung
- 24 -: Gestell
- 25 -: Schlitten
- 26 -: Anpreßvorrichtung
- 27 -: Walze
- 28 -: Walze
- 29 -: Absaugleiste
- 30 -: Verschiebungsrichtung
- 31 -: Nutenplatte
- 32 -: Drainagekanäle
- 33 -: aufgetragener Film
- 34 -: Walzenaufhängung
- 35 -: Absaugnut
- 36 -: Halter
- 37 -: Handkurbel
- 38 -: Rakel
- 39 -: Wanne
- 40 -: Schöpfwalze
- 41 -: Absaugbohrung
- 42 -: Antrieb Nutenplatte
- 43 -: Klemmleisten
- 44 -: Schöpfstation
- 45 -: kontinuierliche Vorrichtung
- 46 -: Durchgangsbohrung in Absaufleiste
- 47 -: Poren/Kanäle in kompressibler Membran
- 48 -: Poren/Kanäle in poröser Trägermembran

## Patentansprüche

1. Verfahren zur Einkapselung von Stoffen in Liposomen (1), insbesondere zur Wirkstoffeinkapselung in Liposomen (1) einer aus monomolekularen Schichten (3, 4) gebildeten Bischicht (11),
**dadurch gekennzeichnet, dass**
zwei unterschiedliche Monoschichten (3, 4) auf die Oberfläche einer porösen Trägermembran (2) aufgebracht werden, wobei die beiden Monoschichten (3, 4) aufgrund physikalischer Wechselwirkungen zwischen den Substanzen der Monoschichten (3, 4) eine Bischicht (11) mit frei einstellbarer Komposition der inneren und äußeren Seite bilden,
auf den jeweiligen Außenseiten der die Bischicht (11) bildenden Monoschichten (3, 4) unterschiedliche Fluidvolumina (5, 7, 10) in Form vorzugsweise dünner Filme (33) aufgebracht werden, die nach der Herstellung der Liposomen (1) die Außenseite der hergestellten Liposomen (1) umgeben und/oder in das Innere der Liposomen (1) eingelagert werden,
eine kompressible Membran (6) derart mit der Schichtenanordnung aus Bischicht (11), Fluidvolumina (5, 7, 10) und poröser Trägermembran (2) in Verbindung gebracht wird, dass in der kompressiblen Membran (6) angeordnete Poren oder Kanäle (24), gefüllt mit einem Puffermedium (7), angrenzend an das in das Innere der Liposomen (1) einzulagernde Fluidvolumen (5, 9) zu liegen kommen,
die Bischicht (11) im Bereich der Poren (48) der porösen Trägermembran (2) durch einen im Puffermedium (7) einzustellenden Überdruck (8) lokal derart deformiert wird, dass die Bischicht (11) in die Poren (23) der porösen Trägermembran (2) hineingedrückt wird und sich, das in das Innere der Liposomen (1) einzulagernde Fluidvolumen (5, 9) mindestens teilweise mit sich reißend, einschnürt und von der Bischicht (11) abreißt,
der abgerissene Bereich (12) der Bischicht (11) sich aufgrund der Minimierung der Grenzflächenenergie zu einem Liposom (1) zusammenschnürt und dabei das mitgerissene Fluidvolumen (5, 9) der in das Innere der Liposomen (1) einzulagernden Substanz (9) einhüllt,
das Liposom (1) durch die poröse Trägermembran (2) hindurch tritt.

2. Verfahren gemäß Anspruch 1, **dadurch gekennzeichnet, dass** die poröse Trägermembran (2) Poren bzw. Kanäle (48) mit exakter Porengröße aufweist.

3. Verfahren gemäß Anspruch 1, **dadurch gekennzeichnet, dass** als poröse Trägermembran (2) eine Filtermembran mit höherer Porosität verwendet wird.

4. Verfahren gemäß einem der vorstehenden Ansprüche, **dadurch gekennzeichnet, dass** die Bischicht (11) sich aufgrund von Wechselwirkungskräften, insbesondere von van der Waals-Kräfte zwischen den Monoschichten (3, 4) bildet.

5. Verfahren gemäß einem der vorstehenden Ansprüche, **dadurch gekennzeichnet, dass** der Überdruck (8) in dem Puffermedium (7) durch Kompression der kompressiblen Membran (6) aufgebaut wird.

6. Verfahren gemäß einem der vorstehenden Ansprüche, **dadurch gekennzeichnet, dass** die poröse Trägermembran (2) aus einer Polycarbonat-Membran (PC-Membran) gebildet wird.

7. Verfahren gemäß einem der vorstehenden Ansprüche, **dadurch gekennzeichnet, dass** die poröse Trägermembran (2) durchgängige Poren oder Kanäle (48) aufweist.

8. Verfahren gemäß Anspruch 7, **dadurch gekennzeichnet, dass** die poröse Trägermembran (2) eine Porosität mit hoher Porendichte, vorzugsweise bis 25% der Oberfläche der Trägermembran (2) aufweist.

9. Verfahren gemäß einem der Ansprüche 7 oder 8, **dadurch gekennzeichnet, dass** die poröse Trägermembran (2) mit Hilfe nanotechnologischer Verfahren, vorzugsweise mit Hilfe der track-etch-Technik hergestellt wird.

10. Verfahren gemäß einem der Ansprüche 7 bis 9, **dadurch gekennzeichnet, dass** die durchgängigen Poren oder Kanäle (48) einen Durchmesser von 10 nm bis zu 20 µm aufweisen.

11. Verfahren gemäß einem der vorstehenden Ansprüche, **dadurch gekennzeichnet, dass** die Größe der hergestellten Liposomen (1) im Bereich zwischen 10 nm bis zu 20 µm beträgt.

12. Verfahren gemäß einem der vorstehenden Ansprüche, **dadurch gekennzeichnet, dass** die Bischicht (11) aufgrund der hohen Porendichte der porösen Trägermembran (2) nahezu vollständig in kugelförmige Liposomen (1) umgewandelt wird.

13. Verfahren gemäß einem der vorstehenden Ansprüche, **dadurch gekennzeichnet, dass** die in den Liposomen (1) innen liegende Monoschicht (4) der Bischicht (11) derart ausgebildet wird, dass die in den Liposomen (1) einzuhüllenden Stoffe an dieser Innenschicht (4) adsorbieren.

14. Verfahren gemäß einem der vorstehenden Ansprüche, **dadurch gekennzeichnet, dass** die in den Liposomen (1) außen liegende Oberflächenschicht (3) der Bischicht (11) derart ausgebildet wird, dass die die Liposomen (11) umhüllenden Stoffe an dieser Außenschicht (3) adsorbieren oder in diese eingebettet werden.

15. Verfahren gemäß Anspruch 14, **dadurch gekennzeichnet, dass** die die Liposomen (1) umhüllenden Stoffe an dieser Außenschicht (3) als sterische Schutzschichten oder Rezeptoren aufgebracht werden.

16. Verfahren gemäß einem der vorstehenden Ansprüche, **dadurch gekennzeichnet, dass** die hergestellten Liposomen (1) mittels eines Drainagesystems (32, 41, 46) aus der durch die Poren bzw. Kanäle (48) der porösen Trägermembran (2) hindurch tretenden Suspension abgesaugt werden.

17. Verfahren gemäß einem der vorstehenden Ansprüche, **dadurch gekennzeichnet, dass** die Fluidvolumina (5, 7, 10) in Form sehr dünner Fluidfilme auf die Bischicht (11) aufgebracht bzw. an der Bischicht (11) angelagert werden.

18. Verfahren gemäß Anspruch 17, **dadurch gekennzeichnet, dass** die Fluidvolumina (5, 7, 10) Schichtdicken bis zu 1 µm aufweisen.

19. Verfahren gemäß einem der vorstehenden Ansprüche, **dadurch gekennzeichnet, dass** das Verfahren kontinuierlich mittels einer Anordnung sich aufeinander abwälzender Walzen (21, 22) ausgeführt wird, auf denen die verschiedenen Membranen (2, 6) und Flüssigkeitsvolumina (5, 7, 10, 3, 4) aufgespannt bzw. aufgeschichtet und durch Abwälzen der verschiedenen Walzen (14, 15, 16, 17, 18) auf den Walzen (21 bzw. 22) miteinander in Kontakt gebracht werden.

20. Verfahren gemäß einem der Ansprüche 1 bis 19, **dadurch gekennzeichnet, dass** das Verfahren diskontinuierlich mittels einer Anordnung von Walzen (27, 28) und einem planaren Träger (31) ausgeführt wird, auf dem die verschiedenen Membranen (2, 6) und Flüssigkeitsvolumina (5, 7, 10, 3, 4) aufgespannt und/oder durch Abwälzen der Walzen (27, 28) miteinander in Kontakt gebracht werden.

21. Diskontinuierlich arbeitende Vorrichtung (23) zur Einkapselung von Stoffen in Liposomen (1), insbesondere zur Durchführung eines Verfahrens gemäß Anspruch 1,
**dadurch gekennzeichnet, dass**
die Vorrichtung (23) einen planaren Träger (31) aufweist, auf dem eine poröse Trägermembran (2) aufgespannt ist und mindestens eine Walze (27, 28) nacheinander die verschiedenen Membranen (2, 6) und Fluidvolumina (5, 7, 10, 3, 4), die auf der mindestens einen Walze (27, 28) vorab auftragbar sind, durch eine Relativbewegung (30) auf der porösen Trägermembran (2) abwälzend mit der porösen Trägermembran (2) in Kontakt bringt.

22. Vorrichtung (23) gemäß Anspruch 21, **dadurch gekennzeichnet, dass** der planare Träger (31) eine Nutenplatte (31) mit Drainagekanälen (32) aufweist.

23. Vorrichtung (23) gemäß einem der Ansprüche 21 oder 22, **dadurch gekennzeichnet, dass** der planare Träger (31) eine dünne Sinterplatte, vorzugsweise aus Polyethylen oder Edelstahl aufweist.

24. Vorrichtung (23) gemäß einem der Ansprüche 21 bis 23, **dadurch gekennzeichnet, dass** die poröse Trägermembran (2) auf der Schichtung aus Nutenplatte (31) mit Drainagekanälen (32) und dünner Sinterplatte aufgebracht ist.

25. Vorrichtung (23) gemäß einem der Ansprüche 21 bis 24, **dadurch gekennzeichnet, dass** eine kompressible Membran (6) auf der mindestens einen Walze (27, 28) aufgebracht ist und mit einstellbarem Druck (26) relativ zu dem planaren Träger (31) verschiebbar und dabei auf dem planaren Träger (31) abwälzbar ist.

26. Vorrichtung (23) gemäß einem der Ansprüche 21 bis 25, **dadurch gekennzeichnet, dass** für jeden der Beschichtungs- und Bearbeitungsvorgänge eine separate Übertragungswalze (28) vorsehbar ist, die nacheinander in die Vorrichtung (23) einbringbar und mit der porösen Trägermembran (2) oder der Walze (28) in Kontakt bringbar sind.

27. Vorrichtung (23) gemäß einem der Ansprüche 21 bis 26, **dadurch gekennzeichnet, dass** die Relativbewegung (30) zwischen dem planaren Träger (31) und der mindestens einen Walze (27, 28) schlupffrei erfolgt.

28. Vorrichtung gemäß einem der Ansprüche 21 bis 27, **dadurch gekennzeichnet, dass** die Walzen (27, 28) die auf ihnen aufgetragenen Schichten (5, 7, 10, 3, 4) entweder auf die Walze (27, 28) oder den planaren Träger (31) abgeben.

29. Vorrichtung (23) gemäß einem der Ansprüche 21 bis 28, **dadurch gekennzeichnet, dass** die Drainage der hergestellten Liposomen (1) durch eine lokale Absaugung nur desjenigen Bereichs des planaren Trägers (31) erfolgt, in dem die Walze (27, 28) und die poröse Trägermembran (2) beim Abwälzen der Walze (27, 28) jeweils aufeinander gepreßt werden.

30. Vorrichtung (23) gemäß Anspruch 29, **dadurch gekennzeichnet, dass** zur Drainage nur derjenige Abschnitt des planaren Trägers (31) mit einer feststehenden Absaugung (29, 46) in wirkende Verbindung gebracht wird, in dem die Walze (27) und die poröse Trägermembran (2) beim Abwälzen der Walze (27, 28) jeweils aufeinander gepreßt werden.

31. Kontinuierlich arbeitende Vorrichtung (45) zur Einkapselung von Stoffen in Liposomen (1), insbesondere zur Durchführung eines Verfahrens gemäß Anspruch 1,
**dadurch gekennzeichnet, dass**
die Vorrichtung (45) einen walzenförmigen Träger (22) aufweist, auf dem eine poröse Trägermembran (2) aufgespannt ist und eine Anordnung sich aufeinander abwälzender Walzen (14, 15, 16, 17, 18, 21) die verschiedenen Membranen (6) und Fluidvolumina (5, 7, 10, 3, 4) durch rotatorische Relativbewegungen (19) auf der porösen Trägermembran (2) abwälzend mit der porösen Trägermembran (2) in Kontakt bringt.

32. Vorrichtung (45) gemäß Anspruch 31, **dadurch gekennzeichnet, dass** der walzenförmige Träger (22) mit der porösen Trägermembran (2) auf einem ebenfalls walzenförmigen Träger (21) mit einer kompressiblen Membran (6) abwälzt.

33. Vorrichtung (45) gemäß einem der Ansprüche 31 oder 32, **dadurch gekennzeichnet, dass** die Beschichtung der sich aufeinander abwälzenden Walzen (14, 15, 16, 17, 18, 21, 22) mit den verschiedenen Fluidvolumina (5, 7, 10, 3, 4) gleichzeitig aufgrund der räumlichen Zuordnung der Walzen (14, 15, 16, 17, 18, 21, 22) zueinander erfolgt.

34. Vorrichtung (45) gemäß einem der Ansprüche 31 bis 33, **dadurch gekennzeichnet, dass** die Relativbewegung zwischen den Walzen (14, 15, 16, 17, 18, 21, 22) schlupffrei mit gleicher Umfangsgeschwindigkeit erfolgt.

35. Vorrichtung (45) gemäß einem der Ansprüche 31 bis 34, **dadurch gekennzeichnet, dass** die zeitliche Abstimmung der Auftragsprozesse auf die Walzen (14, 15, 16, 17, 18, 21, 22) sowie der notwendigen Prozesse zur Trocknung von Schichten (5, 7, 10, 3, 4) bzw. zur Schichtdickenverringerung auf den Walzen (14, 15, 16, 17, 18, 21, 22) durch Variation der Walzendurchmesser bei gleichbleibender Umfangsgeschwindigkeit vornehmbar ist.

36. Vorrichtung (23, 45) gemäß einem der Ansprüche 21 bis 35, **dadurch gekennzeichnet, dass** die Beschichtung der Walze (14, 15, 16, 17, 18, 21, 22, 27, 28) mit den verschiedenen Fluidvolumina (5, 7, 10, 3, 4) durch eine Anordnung aus Schöpfwalzen (13, 40), Rakeln (38) und Walzen (14, 15, 16, 17, 18) durchführbar ist, mit der die Schichtdicke der aufgetragenen Fluidvolumina (5, 7, 10, 3, 4) gezielt einstellbar ist.

37. Vorrichtung (23, 45) gemäß einem der Ansprüche 21 bis 36, **dadurch gekennzeichnet, dass** die die Monoschichten (3, 4) bildenden Substanzen als Lösung in einem Lösungsmittel, insbesondere Ethanol, auf die Walze (17, 18, 28) übertragbar sind.

38. Vorrichtung (23, 45) gemäß Anspruch 37, **dadurch gekennzeichnet, dass** die die Monoschichten (3, 4) bildenden Substanzen nach dem Übertragen auf die Walze (17, 18, 28) auf der Walze (17, 18, 28) trocknen und als getrocknete Filme (33) auf die auf die Membranen (2, 6) aufgetragenen Fluidvolumina (5, 7, 10) übertragbar sind.

39. Vorrichtung (23, 45) gemäß Anspruch 38, **dadurch gekennzeichnet, dass** die auf die Membranen (2, 6) aufgetragenen Fluidvolumina (5, 7, 10) die getrockneten Filme (33) wieder anlösen und auf den Fluidvolumina (5, 10) als Monoschichten (3, 4) ablagern.

40. Vorrichtung (23, 45) gemäß einem der Ansprüche 21 bis 39, **dadurch gekennzeichnet, dass** die Schichtdicke der aufgetragenen Fluidvolumina (5, 7, 10, 3, 4) durch Verdunstung beeinflussbar ist.

41. Vorrichtung (23, 45) gemäß einem der Ansprüche 21 bis 40, **dadurch gekennzeichnet, dass** durch Variation der Elastizität der Beschichtung der Walze (27, 21) mit der kompressiblen Membran (6) und des Anpressdrucks zwischen dieser Walze (27, 21) und der porösen Trägermembran (2) die Druckverteilung in den Poren (23) der porösen Trägermembran (2) bei der Herstellung der Liposomen (1) beeinflussbar ist.

## Claims

1. A method of encapsulating substances in liposomes (1), in particular for active substance encapsulation in liposomes (1) of a bilayer (11) formed from monomolecular layers (3, 4),
**characterised in that**
two different monolayers (3, 4) are applied to the surface of a porous carrier membrane (2), wherein the two monolayers (3, 4) by virtue of physical interactions between the substances of the monolayers (3, 4) form a bilayer (11) of a freely adjustable composition on the inner and outer sides,
applied to the respective outsides of the monolayers (3, 4) forming the bilayer (11) are different fluid volumes (5, 7, 10) in the form of preferably thin films (33) which, after production of the liposomes (1) enclose the outside of the produced liposomes (1) and/or are incorporated into the interior of the liposomes (1),
a compressible membrane (6) is brought into relationship with the layer arrangement of the bilayer (11), fluid volumes (5, 7, 10) and porous carrier membrane (2) in such a way that pores or passages (24) arranged in the compressible membrane (6), filled with a buffer medium (7), come to lie in adjacent relationship with the fluid volumes (5, 9) to be incorporated into the interior of the liposomes (1),
the bilayer (11) in the region of the pores (48) of the porous carrier membrane (2) is locally deformed by an increased pressure (8) to be set in the buffer medium (7) in such a way that the bilayer (11) is urged into the pores (23) of the porous carrier membrane (2) and, at least partially pulling with itself the fluid volume (5, 9) to be incorporated into the interior of the liposomes (1), contracts and tears away from the bilayer (11),
the torn-away region (12) of the bilayer (11) binds by virtue of the minimisation of the interfacial energy to a liposome (1) and in so doing encases the entrained fluid volume (5, 9) of the substance (9) to be incorporated into the interior of the liposomes (1), and
the liposome (1) passes through the porous carrier membrane (2).

2. A method according to claim 1 **characterised in that** the porous carrier membrane (2) has pores or passages (48) of exact pore size.

3. A method according to claim 1 **characterised in that** a filter membrane with a higher level of porosity is used as the porous carrier membrane (2).

4. A method according to one of the preceding claims **characterised in that** the bilayer (11) is formed by virtue of interaction forces, in particular van der Waals forces between the monolayers (3, 4).

5. A method according to one of the preceding claims **characterised in that** the increased pressure (8) in the buffer medium (7) is built up by compression of the compressible membrane (6).

6. A method according to one of the preceding claims **characterised in that** the porous carrier membrane (2) is formed from a polycarbonate membrane (PC membrane).

7. A method according to one of the preceding claims **characterised in that** the porous carrier membrane (2) has continuous pores or passages (48).

8. A method according to claim 7 **characterised in that** the porous carrier membrane (2) has a porosity of high pore density, preferably up to 25% of the surface area of the carrier membrane (2).

9. A method according to one of claims 7 and 8 **characterised in that** the porous carrier membrane (2) is produced by means of nanotechnology methods, preferably by means of the track-etch procedure.

10. A method according to one of claims 7 to 9 **characterised in that** the continuous pores or passages (48) are of a diameter of 10 nm to 20 µm.

11. A method according to one of the preceding claims **characterised in that** the size of the liposomes (1) produced is in the range of between 10 nm and 20 µm.

12. A method according to one of the preceding claims **characterised in that** the bilayer (11) is converted almost completely into spherical liposomes (1) by virtue of the high pore density of the porous carrier membrane (2).

13. A method according to one of the preceding claims **characterised in that** the monolayer (4) of the bilayer (11), that is disposed inwardly in the liposomes (1), is of such a nature that the substances to be encased in the liposomes (1) adsorb to said internal layer (4).

14. A method according to one of the preceding claims **characterised in that** the surface layer (3) of the bilayer (11), that is disposed outwardly in the liposomes (1), is of such a nature that the substances encasing the liposomes (1) adsorb to or are embedded in said external layer (3).

15. A method according to claim 14 **characterised in that** the substances encasing the liposomes (1) are applied to said external layer (3) as stearic protective layers or receptors.

16. A method according to one of the preceding claims **characterised in that** the liposomes (1) produced are sucked by means of a drainage system (32, 41, 46) out of the suspension passing through the pores or passages (48) of the porous carrier membrane (2).

17. A method according to one of the preceding claims **characterised in that** the fluid volumes (5, 7, 10) are applied in the form of very thin fluid films to the bilayer (11) or are deposited at the bilayer (11).

18. A method according to claim 17 **characterised in that** the fluid volumes (5, 7, 10) involve layer thicknesses of up to 1 µm.

19. A method according to one of the preceding claims **characterised in that** the method is carried out continuously by means of an arrangement of rollers (21, 22) which roll against each other and on which the various membranes (2, 6) and fluid volumes (5, 7, 10, 3, 4) are spread and coated respectively and are brought into contact with each other by rolling of the various rollers (14, 15, 16, 17, 18) against the rollers (21 and 22 respectively).

20. A method according to one of claims 1 to 19 **characterised in that** the method is carried out discontinuously by means of an arrangement of rollers (27, 28) and a planar carrier (31) on which the various membranes (2, 6) and fluid volumes (5, 7, 10, 3, 4) are spread and/or brought into contact with each other by rolling of the rollers (27, 28).

21. A discontinuously operating apparatus (23) for the encapsulation of substances in liposomes (1), in particular for carrying out a method according to claim 1,
**characterised in that**
the apparatus (23) has a planar carrier (31) on which a porous carrier membrane (2) is spread and at least one roller (27, 28) successively brings the various membranes (2, 6) and fluid volumes (5, 7, 10, 3, 4) which can be previously applied to the at least one roller (27, 28) into contact with the porous carrier membrane (2) in rolling relationship by a relative movement (30) on the porous carrier membrane (2).

22. Apparatus (23) according to claim 21 **characterised in that** the planar carrier (31) has a grooved plate (31) with drainage passages (32).

23. Apparatus (23) according to one of claims 21 and 22 **characterised in that** the planar carrier (31) has a thin sintered plate, preferably of polyethylene or high-quality steel.

24. Apparatus (23) according to one of claims 21 to 23 **characterised in that** the porous carrier membrane (2) is applied to the layer arrangement consisting of the grooved plate (31) with drainage passage (32) and the thin sintered plate.

25. Apparatus (23) according to one of claims 21 to 24 **characterised in that** a compressible membrane (6) is applied to the at least one roller (27, 28) and is displaceable with an adjustable pressure (26) relative to the planar carrier (31) and **in that** situation can be rolled on the planar carrier (31).

26. Apparatus (23) according to one of claims 21 to 25 **characterised in that** a separate transfer roller (28) can be provided for each of the coating and working operations, which rollers can be successively introduced into the apparatus (23) and brought into contact with the porous carrier membrane (2) or the roller (28).

27. Apparatus (23) according to one of claims 21 to 26 **characterised in that** the relative movement (30) between the planar carrier (31) and the at least one roller (27, 28) is effected in slip-free manner.

28. Apparatus (23) according to one of claims 21 to 27 **characterised in that** the rollers (27, 28) deliver the layers (5, 7, 10, 3, 4) applied to them either on to the roller (27, 28) or the planar carrier (31).

29. Apparatus (23) according to one of claims 21 to 28 **characterised in that** drainage of the liposomes (1) produced is effected by locally sucking away only that region of the planar carrier (31), in which the roller (27, 28) and the porous carrier membrane (2) are respectively pressed against each other in the rolling movement of the roller (27, 28).

30. Apparatus (23) according to claim 29 **characterised in that** for the drainage operation only that portion of the planar carrier (31) in which the roller (27) and the porous carrier membrane (2) are respectively pressed against each other in the rolling movement of the roller (27, 28) is brought into operative relationship with a stationary suction removal means (29, 46).

31. A continuously operating apparatus (45) for the encapsulation of substances in liposomes (1), in particular for carrying out a method according to claim 1,
**characterised in that**
the apparatus (45) has a carrier (22) in roller form, on which a porous carrier membrane (2) is spread, and an arrangement of rollers (14, 15, 16, 17, 18, 21) which roll against each other brings the various membranes (6) and fluid volumes (5, 7, 10, 3, 4) into contact with the porous carrier membrane (2) in rolling relationship by rotational relative movements (19) on the porous carrier membrane (2).

32. Apparatus (45) according to claim 31 **characterised in that** the carrier (22) in roller form rolls with the porous carrier membrane (2) on a carrier (21) which is also in roller form with a compressible membrane (6).

33. Apparatus (45) according to one of claims 31 and 32 **characterised in that** coating of the rollers (14, 15, 16, 17, 18, 21, 22) which roll against each other with the various fluid volumes (5, 7, 10, 3, 4) is effected simultaneously by virtue of the spatial arrangement of the rollers (14, 15, 16, 17, 18, 21, 22) relative to each other.

34. Apparatus (45) according to one of claims 31 to 33 **characterised in that** the relative movement between the rollers (14, 15, 16, 17, 18, 21, 22) is effected slip-free at the same peripheral speed.

35. Apparatus (45) according to one of claims 31 to 34 **characterised in that** matching in respect of time of the application processes to the rollers (14, 15, 16, 17, 18, 21, 22) and the necessary processes for drying of layers (5, 7, 10, 3, 4) or for layer thickness reduction on the rollers (14, 15, 16, 17, 18, 21, 22) can be effected by a variation in the roller diameter with the peripheral speed remaining the same.

36. Apparatus (23, 45) according to one of claims 21 to 35 **characterised in that** coating of the rollers (14, 15, 16, 17, 18, 21, 22, 27, 28) with the various fluid volumes (5, 7, 10, 3, 4) can be carried out by an arrangement comprising scoop rollers (13, 40), doctors (38) and rollers (14, 15, 16, 17, 18), with which the layer thickness of the applied fluid volumes (5, 7, 10, 3, 4) can be specifically adjusted.

37. Apparatus (23, 45) according to one of claims 21 to 36 **characterised in that** the substances forming the monolayers (3, 4) can be transferred in the form of a solution in a solvent, in particular ethanol, on to the roller (17, 18, 28).

38. Apparatus (23, 45) according to claim 37 **characterised in that** the substances forming the monolayers (3, 4) after transfer on to the roller (17, 18, 28) dry on the roller (17, 18, 28) and can be transferred as dried films (33) on to the fluid volumes (5, 7, 10) applied to the membranes (2, 6).

39. Apparatus (23, 45) according to claim 38 **characterised in that** the fluid volumes (5, 7, 10) applied to the membranes (2, 6) dissolve the dried films (33) again and are deposited on the fluid volumes (5, 10) in the form of monolayers (3, 4).

40. Apparatus (23, 45) according to one of claims 21 to 39 **characterised in that** the layer thickness of the applied fluid volumes (5, 7, 10, 3, 4) can be influenced by evaporation.

41. Apparatus (23, 45) according to one of claims 21 to 40 **characterised in that** the pressure distribution in the pores (23) of the porous carrier membrane (2) can be influenced in the production of the liposomes (1) by a variation in the elasticity of the coating of the roller (27, 21) with the compressible membrane (6) and the contact pressure between said roller (27, 21) and the porous carrier membrane (2).

## Revendications

1. Procédé d'encapsulation de substances dans des liposomes (1), en particulier d'encapsulation de principes actifs dans des liposomes (1) constitués d'une bicouche (11) formée par des couches monomoléculaires (3, 4),
**caractérisé en ce que**
deux monocouches (3, 4) différentes sont déposées sur la surface d'une membrane support poreuse (2), les deux monocouches (3, 4) formant par le biais d'interactions physiques entre les substances des monocouches (3, 4) une bicouche (11) dont la composition de la face interne et de la face externe peut être ajustée librement,
différents volumes de fluides (5, 7, 10) sont déposés sur les faces externes respectives des monocouches (3, 4) qui forment la bicouche (11), de préférence sous la forme de films minces (33) qui entourent la face externe des liposomes (1) préparés et/ou sont incorporés à l'intérieur des liposomes (1) à l'issue de la préparation des liposomes (1),
une membrane compressible (6) est combinée avec l'agencement de couches constitué de la bicouche (11), des volumes de fluides (5, 7, 10) et de la membrane support poreuse (2) de telle façon que des pores ou canaux (24) ménagés dans la membrane compressible (6) et remplis d'un milieu tampon (7) sont positionnés adjacents au volume de fluide (5, 9) à incorporer à l'intérieur des liposomes (1),
dans la zone des pores (48) de la membrane support poreuse (2), la bicouche (11) est localement déformée par une surpression (8) à créer dans le milieu tampon (7) de telle façon que la bicouche (11) est comprimée dans les pores (23) de la membrane support poreuse (2) et que le volume de fluide (5, 9) à incorporer à l'intérieur des liposomes (1) subit au moins en partie une striction due à l'entraînement et se détache de la bicouche (11),
à cause de la diminution de l'énergie interfaciale, la zone détachée (12) de la bicouche (11) se resserre en un liposome (1) et enveloppe ainsi le volume de fluide entraîné (5, 9) de la substance (9) à incorporer à l'intérieur des liposomes (1),
le liposome (1) traverse la membrane support poreuse (2).

2. Procédé selon la revendication 1, **caractérisé en ce que** la membrane support poreuse (2) présente des pores ou canaux (48) ayant une taille de pore exacte.

3. Procédé selon la revendication 1, **caractérisé en ce que** la membrane support poreuse (2) utilisée est une membrane filtrante de porosité plus élevée.

4. Procédé selon l'une des revendications précédentes, **caractérisé en ce que** la bicouche (11) se forme sous l'action de forces d'interactions, en particulier de forces de van der Waals entre les monocouches (3, 4).

5. Procédé selon l'une des revendications précédentes, **caractérisé en ce que** la surpression (8) dans le milieu tampon (7) est générée par une compression de la membrane compressible (6).

6. Procédé selon l'une des revendications précédentes, **caractérisé en ce que** la membrane support poreuse (2) est formée par une membrane en polycarbonate (membrane en PC).

7. Procédé selon l'une des revendications précédentes, **caractérisé en ce que** la membrane support poreuse (2) présente des pores ou canaux (48) traversants.

8. Procédé selon la revendication 7, **caractérisé en ce que** la membrane support poreuse (2) présente une porosité correspondant à une densité de pores élevée, de préférence allant jusqu'à 25% de l'aire de surface de la membrane support (2).

9. Procédé selon l'une des revendications 7 ou 8, **caractérisé en ce que** la membrane support poreuse (2) est fabriquée par des procédés nanotechnologiques, de préférence par la technique track etch.

10. Procédé selon l'une des revendications 7 à 9, **caractérisé en ce que** les pores ou canaux traversants (48) ont un diamètre allant de 10 nm à 20 µm.

11. Procédé selon l'une des revendications précédentes, **caractérisé en ce que** la taille des liposomes (1) produits se situe dans la plage de 10 nm à 20 µm.

12. Procédé selon l'une des revendications précédentes, **caractérisé en ce que** la bicouche (11), compte tenu de la grande densité de pores de la membrane support poreuse (2), est presque entièrement transformée en liposomes (1) sphériques.

13. Procédé selon l'une des revendications précédentes, **caractérisé en ce que** la monocouche (4) de la bicouche (11) qui se trouve à l'intérieur dans les liposomes (1) est constituée de telle façon que les substances à encapsuler dans les liposomes (1) sont adsorbées sur cette couche interne (4).

14. Procédé selon l'une des revendications précédentes, **caractérisé en ce que** la couche superficielle (3) de la bicouche (11) qui se trouve à l'extérieur dans les liposomes (1) est constituée de telle façon que les substances à encapsuler dans les liposomes (1) sont adsorbées sur, ou incorporées dans, cette couche externe (3).

15. Procédé selon la revendication 14, **caractérisé en ce que** les substances qui enveloppent les liposomes (1) sont déposées sur cette couche externe (3) sous la forme de couches de protection ou de récepteurs stériques.

16. Procédé selon l'une des revendications précédentes, **caractérisé en ce que** les liposomes (1) produits sont aspirés au moyen d'un système de drainage (32, 41, 46) hors de la suspension qui traverse les pores ou canaux (48) de la membrane support poreuse (2).

17. Procédé selon l'une des revendications précédentes, **caractérisé en ce que** les volumes de fluides (5, 7, 10) sont déposés sur la bicouche (11) ou se fixent sur la bicouche (11) sous la forme de films de fluide très minces.

18. Procédé selon la revendication 17, **caractérisé en ce que** les volumes de fluides (5, 7, 10) présentent des épaisseurs de couches allant jusqu'à 1 µm.

19. Procédé selon l'une des revendications précédentes, **caractérisé en ce que** le procédé s'effectue en continu au moyen d'un système de cylindres roulant les uns sur les autres (21, 22), sur lesquels les différentes membranes (2, 6) et les différents volumes de liquide (5, 7, 10, 3, 4) sont tendus ou étalés et mis en contact les uns avec les autres par le roulement des différents cylindres (14, 15, 16, 17, 18) sur les cylindres (21 ou 22).

20. Procédé selon l'une des revendications 1 à 19, **caractérisé en ce que** le procédé s'effectue de manière discontinue au moyen d'un système de cylindres (27, 28) et d'un support plan (31) sur lequel les différentes membranes (2, 6) et les différents volumes de liquide (5, 7, 10, 3, 4) sont tendus et/ou mis en contact les uns avec les autres par le roulement des cylindres (27, 28).

21. Dispositif travaillant de manière discontinue (23) pour encapsuler des substances dans des liposomes (1), en particulier pour mettre en oeuvre un procédé selon la revendication 1,
**caractérisé en ce que**
le dispositif (23) présente un support plan (31) sur lequel une membrane support poreuse (2) est tendue et au moins un cylindre (27, 28) met successivement les différentes membranes (2, 6) et les différents volumes de fluides (5, 7, 10, 3, 4), qui peuvent être déposés au préalable sur l'un au moins des cylindres (27, 28), en contact avec la membrane support poreuse (2) par un mouvement relatif (30) de roulement sur la membrane support poreuse (2).

22. Dispositif (23) selon la revendication 21, **caractérisé en ce que** le support plan (31) présente une plaque rainurée (31) comportant des canaux de drainage (32).

23. Dispositif (23) selon l'une des revendications 21 ou 22, **caractérisé en ce que** le support plan (31) présente une plaque frittée mince, de préférence en polyéthylène ou en acier spécial.

24. Dispositif (23) selon l'une des revendications 21 à 23, **caractérisé en ce que** la membrane support poreuse (2) est déposée sur l'empilage constitué par la plaque rainurée (31) comportant des canaux de drainage (32) et par la place frittée mince.

25. Dispositif (23) selon l'une des revendications 21 à 24, **caractérisé en ce qu'**une membrane compressible (6) est déposée sur au moins un cylindre (27, 28) et peut se déplacer sous une pression réglable (26) par rapport au support plan (31) et rouler ainsi sur ledit support plan (31).

26. Dispositif (23) selon l'une des revendications 21 à 25, **caractérisé en ce que**, pour chacune des opérations de revêtement et de façonnage, il peut être prévu un cylindre de transfert séparé (28) qui peut être introduit successivement dans le dispositif (23) et amené en contact avec la membrane support poreuse (2) ou le cylindre (28).

27. Dispositif (23) selon l'une des revendications 21 à 26, **caractérisé en ce que** le mouvement relatif (30) entre le support plan (31) et l'un au moins des cylindres (27, 28) s'effectue sans glissement.

28. Dispositif selon l'une des revendications 21 à 27, **caractérisé en ce que** les cylindres (27, 28) transfèrent les couches (5, 7, 10, 3, 4) qui ont été déposées sur eux soit sur le cylindre (27, 28), soit sur le support plan (31).

29. Dispositif (23) selon l'une des revendications 21 à 28, **caractérisé en ce que** le drainage des liposomes (1) produits se fait par une aspiration locale uniquement dans la zone du support plan (31) dans laquelle le cylindre (27, 28) et la membrane support poreuse (2) sont comprimés l'un contre l'autre par le roulement du cylindre (27, 28).

30. Dispositif (23) selon la revendication 29, **caractérisé en ce que**, pour le drainage, seule la partie du support plan (31) dans laquelle le cylindre (27) et la membrane support poreuse (2) sont respectivement comprimés l'un contre l'autre par le roulement du cylindre (27, 28) est mise en liaison active avec une aspiration fixe (29, 46).

31. Dispositif travaillant de manière discontinue (45) pour encapsuler des substances dans des liposomes (1), en particulier pour mettre en oeuvre un procédé selon la revendication 1,
**caractérisé en ce que**
le dispositif (45) présente un support cylindrique (22) sur lequel une membrane support poreuse (2) est tendue et un agencement de cylindres roulant les uns sur les autres (14, 15, 16, 17, 18, 21) met les différentes membranes (6) et les différents volumes de fluides (5, 7, 10, 3, 4) en contact avec la membrane support poreuse (2) par des mouvements de rotation relatifs (19) en roulant sur la membrane support poreuse (2).

32. Dispositif (45) selon la revendication 31, **caractérisé en ce que** le support cylindrique (22) portant la membrane support poreuse (2) roule sur un support (21) également cylindrique portant une membrane compressible (6).

33. Dispositif (45) selon l'une des revendications 31 ou 32, **caractérisé en ce que** le dépôt des différents volumes de fluides (5, 7, 10, 3, 4) sur les cylindres (14, 15, 16, 17, 18, 21, 22) roulant les uns sur les autres a lieu simultanément compte tenu de la correspondance spatiale des cylindres (14, 15, 16, 17, 18, 21, 22).

34. Dispositif (45) selon l'une des revendications 31 à 33, **caractérisé en ce que** le mouvement relatif entre les cylindres (14, 15, 16, 17, 18, 21, 22) se fait sans glissement à une vitesse circonférentielle égale.

35. Dispositif (45) selon l'une des revendications 31 à 34, **caractérisé en ce que** l'adaptation dans le temps des processus de dépôt sur les cylindres (14, 15, 16, 17, 18, 21, 22) ainsi que des processus nécessaires de séchage des couches (5, 7, 10, 3, 4) ou de réduction des épaisseurs de couches sur les cylindres (14, 15, 16, 17, 18, 21, 22) peut s'effectuer en faisant varier le diamètre des cylindres à vitesse circonférentielle constante.

36. Dispositif (23, 45) selon l'une des revendications 21 à 35, **caractérisé en ce que** le dépôt des différents volumes de fluides (5, 7, 10, 3, 4) sur les cylindres (14, 15, 16, 17, 18, 21, 22, 27, 28) peut se faire au moyen d'un agencement de cylindres alimentateurs (13, 40), de racles (38) et de cylindres (14, 15, 16, 17, 18) permettant d'ajuster de manière ciblée l'épaisseur de couche des volumes de fluides (5, 7, 10, 3, 4) déposés.

37. Dispositif (23, 45) selon l'une des revendications 21 à 36, **caractérisé en ce que** les substances qui forment les monocouches (3, 4) peuvent être transférées sur le cylindre (17, 18, 28) sous la forme d'une solution dans un solvant, en particulier l'éthanol.

38. Dispositif (23, 45) selon la revendication 37, **caractérisé en ce que**, après avoir été déposées sur le cylindre (17, 18, 28), les substances qui forment les monocouches (3, 4) sèchent sur le cylindre (17, 18, 28) et peuvent être transférées sous la forme de films séchés (33) sur les volumes de fluides (5, 7, 10) déposés sur les membranes (2, 6).

39. Dispositif (23, 45) selon la revendication 38, **caractérisé en ce que** les volumes de fluides (5, 7, 10) déposés sur les membranes (2, 6) dissolvent à nouveau les films séchés (33) et les fixent sous forme de monocouches (3, 4) sur les volumes de fluides (5, 10).

40. Dispositif (23, 45) selon l'une des revendications 21 à 39, **caractérisé en ce que** l'on peut agir par une évaporation sur l'épaisseur de couche des volumes de fluides (5, 7, 10, 3, 4) déposés.

41. Dispositif (23, 45) selon l'une des revendications 21 à 40, **caractérisé en ce que** l'on peut agir sur la distribution de la pression dans les pores (23) de la membrane support poreuse (2) lors de la production des liposomes (1) en faisant varier l'élasticité du revêtement du cylindre (27, 21) portant la membrane (6) compressible et la pression d'appui entre ce cylindre (27, 21) et la membrane support poreuse (2).
